(19) Europäisches Patentamt
European Patent Office
Office européen des brevets

(11) EP 4 268 851 A1

(12) **EUROPEAN PATENT APPLICATION**
published in accordance with Art. 153(4) EPC

(43) Date of publication:
**01.11.2023 Bulletin 2023/44**

(21) Application number: **21911591.2**

(22) Date of filing: **23.12.2021**

(51) International Patent Classification (IPC):
***A61K 48/00*** *(2003.01)* ***A61K 9/51*** *(2006.01)*
***A61K 31/7088*** *(2006.01)* ***A61P 35/00*** *(2006.01)*
***A61K 45/06*** *(2006.01)*

(52) Cooperative Patent Classification (CPC):
**A61K 9/51; A61K 31/7088; A61K 45/06; A61K 48/00; A61P 35/00**

(86) International application number:
**PCT/KR2021/019773**

(87) International publication number:
**WO 2022/139526 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.12.2020 KR 20200183898**
**09.11.2021 KR 20210153381**

(71) Applicant: **EnhancedBio Inc.**
**Seoul 04779 (KR)**

(72) Inventors:
- **LEE, Hyukjin**
  **Seoul 06557 (KR)**
- **JEONG, Michaela**
  **Seoul 02531 (KR)**
- **KIM, Minjeong**
  **Seoul 06544 (KR)**
- **KIM, Hong Joong**
  **Seoul 04779 (KR)**
- **JUNG, Hun Soon**
  **Seoul 04779 (KR)**
- **KIM, Hye Jeong**
  **Seoul 04779 (KR)**
- **KIM, Yi Sak**
  **Seoul 04779 (KR)**
- **KANG, Ha Rim**
  **Seoul 04779 (KR)**

(74) Representative: **Bates, Philip Ian**
**Reddie & Grose LLP**
**The White Chapel Building**
**10 Whitechapel High Street**
**London E1 8QS (GB)**

Remarks:
The complete document including Reference Table(s) and the Sequence Listing(s) can be downloaded from the EPO website

(54) **COMPOSITION FOR PREVENTING OR TREATING CANCER, CONTAINING LIPID NANOPARTICLES**

(57) The present application relates to a composition for preventing or treating cancer, containing lipid nanoparticles. A pharmaceutical composition according to one embodiment has excellent biocompatibility, can deliver gene therapeutic agents and the like with high efficiency, thereby having excellent cancer prevention or treatment effects, and has excellent cancer prevention or treatment effects even if used in combination with anticancer agents and/or radiation therapy.

【FIG. 5a】

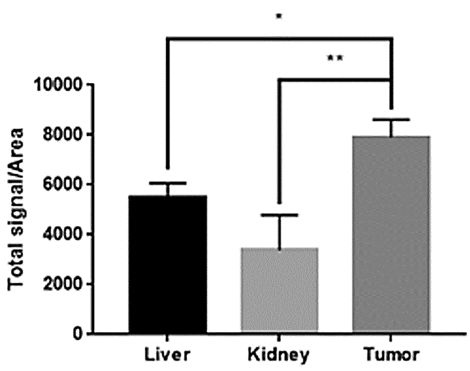


EP 4 268 851 A1

## Description

[TECHNICAL FIELD]

The present invention relates to a composition for preventing or treating cancer comprising a lipid nanoparticle.

[BACKGROUND ART]

In the pharmaceutical formulation industry, the drug delivery system (DDS), designed to efficiently deliver the required amount of the drug by reducing side effects of the drug and maximizing efficacy and effects, is a high value-added core technology which can create economic benefits comparable to that of new drug development and has great potential for success and its purpose is to improve the quality of patient treatment by making drug administration more efficient. The solubilization technology of poorly soluble drugs belonging to the drug absorption promotion technology, which is one of the core technologies of the drug delivery system, is considered the most reasonable way to reduce the development cost of new drug substances and at the same time increase the added value of currently marketed drugs. In particular, the development of improved new drugs through the development of drug solubilization technology in a situation where new drug development conditions are poor as in Korea is a field that can create enormous added value at a low cost.

Gene therapy using a genetic drug delivery system is established in a large hope of modifying genetic binding and to treat numerous diseases. In the successful and securely performing this gene therapy, the effective gene delivery is one of the main challenges and the virus delivery system was proved to be effective in gene delivery. However, due to some defects such as immunogenicity, limitation of the inserted DNA size and difficulties of mass production, the use of viruses are limited as a gene delivery system. Non-viral gene carriers such as cationic liposome and polymers began to be noted as an alternative means of a viral system.

Improved stability profile and ease of manufacturing and operation of the polymer delivery system triggered studies on the design and synthesis of a non-toxic and biodegradable polymer carrier for effective and safe gene delivery. Poly(L-lysine), polyethylenimine, starburst, polyamidoamine dendrimer, and cationic liposome voluntarily, and the like can be self-assembled and compress plasmid DNA (pDNA) into a small structure sufficiently to enter cells through endocytosis, and therefore they have been widely studied as a non-viral gene delivery system.

Nucleic acids such as antisense RNA, siRNA, and the like are a material capable of inhibiting expression of specific proteins in vivo, and are spotlighted as an important tool for treatment of cancer, genetic diseases, infectious diseases, autoimmune diseases, and the like (Novina and Sharp, Nature, 430, 161-164, 2004). However, nucleic acids such as siRNA are difficult to deliver directly into cells and they are easily decomposed by enzymes in the blood, so there are many studies to overcome them. To date, the method for delivering nucleic acids into cells, a method for carrying by mixing with a positive charge lipid or polymer (named lipid-DNA conjugate (lipoplex) and polymer-DNA conjugate (polyplex), respectively) is mainly used (Hirko et al., Curr. Med. Chem., 10, 1185-93, 2003; Merdan et al., Adv. Drug. Deliv. Rev., 54, 715-58, 2002; Spagnou et al., Biochemistry, 43, 13348-13356, 2004). The lipid-DNA conjugate is combined with the nucleic acid to deliver the nucleic acid well to cells and thus it is used a lot at the cell level, but in vivo, when injecting locally, in many cases, it has a disadvantage of inducing inflammation in the body (Filonand and Phillips, Biochim. Biophys. Acta, 1329, 345-56, 1997), and accumulating it in tissues such as lung, liver, spleen and the like which are mainly primary passage organs during intravascular injection (Ren et al., Gene Therapy, 7, 764-768, 2000).

In addition, such a non-viral delivery system has a problem of low transfection efficiency. Many efforts have been tilted to enhance transfection efficiency, but this is still far from the system that is stable. In addition, the carrier of the non-viral gene delivery system represents a significantly high cytotoxicity due to poor biocompatibility and non-biodegradability.

Under such a technical background, there is a need to develop a nanoparticle with an excellent anticancer effect, as it can efficiently deliver an anticancer agent to a target organ or cell.

[DISCLOSURE]

[TECHNICAL PROBLEM]

One embodiment provides a pharmaceutical composition for preventing or treating cancer, comprising a lipid nanoparticle comprising an ionizable lipid in which a 6-membered heterocyclic amine and alkyl-epoxide are bonded; a phospholipid; and a lipid-PEG (polyethyleneglycol) conjugate and

(2) a first anticancer agent, and
wherein the first anticancer agent is an anionic drug, a nucleic acid, or a combination thereof.

Another embodiment provides a pharmaceutical composition for treating cancer for use in combination with radiation therapy comprising the pharmaceutical composition.

Other embodiment provides a pharmaceutical composition for use in combination of an anticancer agent, further comprising the pharmaceutical composition and a second anticancer agent.

[TECHNICAL SOLUTION]

One aspect to achieve the above objects, provides a pharmaceutical composition for preventing or treating cancer, comprising (1) a lipid nanoparticle comprising an ionizable lipid in which a 6-membered heterocyclic amine and alkyl-epoxide are bonded; a phospholipid; cholesterol; and a lipid-PEG (polyethyleneglycol) conjugate and

(2) a first anticancer agent,
wherein the first anticancer agent is an anionic drug, a nucleic acid, or a combination thereof.

The lipid nanoparticle comprised in the pharmaceutical composition has excellent biocompatibility, and can deliver an anticancer agent (for example, gene therapeutic agent) and the like with high efficiency, and can be usefully used in related technical fields such as lipid nanoparticle-mediated gene therapy and imaging diagnosis technology.

Herein, 'ionizable lipid' or 'lipidoid' mean an amine-containing lipid which can be easily protonated, and for example, it may be a lipid of which charge state changes depending on the surrounding pH. The ionizable lipid may be one in which a 6-membered heterocyclic amine and alkyl-epoxide are combined. Specifically, the ionizable lipid may be a compound having characteristics similar to the lipid produced by reaction of the 6-membered heterocyclic amine and alkyl-epoxide, and more specifically, it may be a compound produced by ring opening reaction of epoxide by reacting the 6-membered heterocyclic amine with alkyl-epoxide.

In one embodiment, the ionizable lipid may be one in which a 6-membered heterocyclic amine and alkyl-epoxide are combined by reacting them at a molar ratio of 1:n (n = number of primary amines comprised in 6-membered heterocyclic amine x 2 + number of secondary amines x 1). According to one specific embodiment, it may be prepared by mixing 246 amine and 1,2-epoxydodecane at a molar ratio of 1 : 4 and reacting them under the conditions of 700 to 800rpm and 85 to 95 for 2 to 4 days.

The ionizable lipid may be protonated (positively charged) at a pH below the pKa of a cationic lipid, and it may be substantially neutral at a pH over the pKa. In one embodiment, the lipid nanoparticle may comprise a protonated ionizable lipid and/or an ionizable lipid showing neutrality.

The ionizable lipid is an ionizable compound having characteristics similar to the lipid, and through electrostatic interaction with a drug (for example, anionic drug and/or nucleic acid), may play a role of encapsulating the drug within the lipid nanoparticle with high efficiency.

The 6-membered heterocyclic amine may comprise a structure of piperazine or piperidine.

The 6-membered heterocyclic amine may be a chain or non-chain amine comprising a tertiary amine, and according to one embodiment, it may be one or more kinds selected from the group consisting of

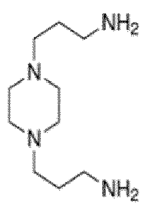,
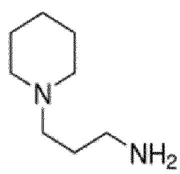,
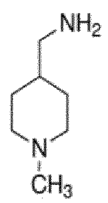,
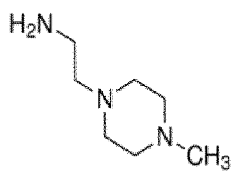,

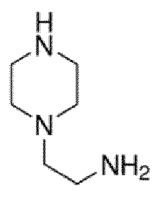, and
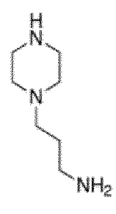.

In one embodiment, the 6-membered heterocyclic amine may be one or more kinds selected from the group consisting of 1,4-bis(3-aminopropyl)piperazine, N-(3-Aminopropyl)piperidine, (1-Methyl-4-piperidinyl)methanamine, 2-(4-Methyl-piperazin-1-yl)-ethylamine, 1-(2-Aminoethyl)piperazine, and 1-(3-aminopropyl)piperazine.

According to the type of the amine comprised in the ionizable lipid, (i) the drug encapsulation efficiency, (ii) PDI

(polydispersity index), and/or (iii) the drug delivery efficiency of the pharmaceutical composition to cancer tissue and/or cancer cells of the lipid nanoparticle may be different.

The lipid nanoparticle comprising an ionizable lipid comprising an amine may have one or more kinds of the following characteristics:

(1) encapsulating a drug into the lipid nanoparticle with high efficiency;
(2) uniform size of the prepared lipid nanoparticle (or having a low PDI value); and/or
(3) excellent drug delivery efficiency to cancer tissue, and/or cancer cells;
(4) capability of long circulation in vivo; and/or
(5) excellent cancer prevention and/or treatment effects.

According to one embodiment, the lipid nanoparticle comprising an ionizable lipid comprising 1,4-bis(3-aminopropyl)piperazine (Cas Nos. 7209-38-3) may have one or more kinds of the following characteristics than that comprising an ionizable lipid comprising other types of amines:

(1) encapsulating a drug into the lipid nanoparticle with high efficiency;
(2) uniform size of the prepared lipid nanoparticle (or having a low PDI value); and/or
(3) excellent drug delivery efficiency to cancer tissue, and/or cancer cells;
(4) capability of long circulation in vivo; and/or
(5) excellent cancer prevention and/or treatment effects.

The alkyl-epoxide may have the structure of Chemical formula 1 below.

[Chemical formula 1]

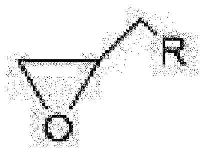

The alkyl-epoxide may have a carbon length of C6 to C14, C6 to C12, C6 to C10, C8 to C14, C8 to C12, C8 to C10, C10 to C14, C10 to C12, or C10, and for example, it may be 1,2-epoxydodecane of C10. By setting the carbon number of the alkyl-epoxide comprised in the ionizable lipid to the above range, it is possible to represent a high encapsulation efficiency for the drug encapsulated in the lipid nanoparticle.

In one embodiment, the ionizable lipid may have the general formula of Chemical formula 2 below.

[Chemical formula 2]

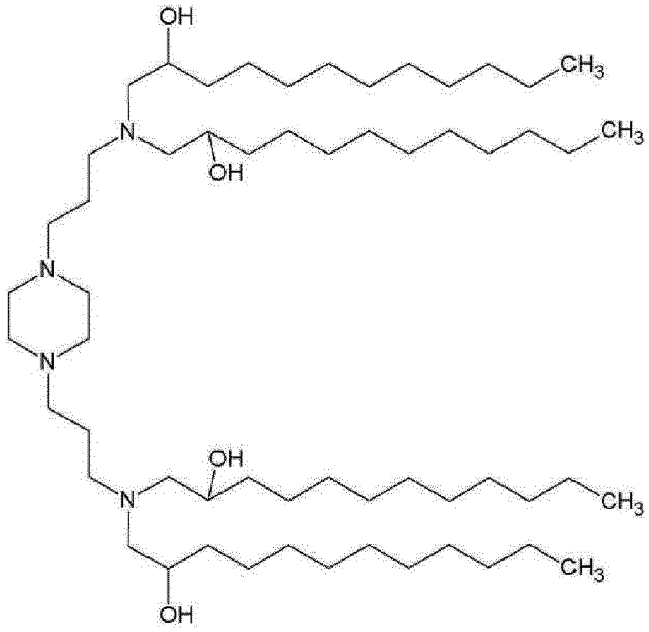

The structure of Chemical formula 2 is one example of the structure of the ionizable lipid according to one embodiment, and the structure of the ionizable lipid may be different depending on the type of the 6-membered heterocyclic amine and alkyl-epoxide.

According to one embodiment, a lipid nanoparticle comprising an ionizable lipid having the structure of Chemical formula 2 may have one or more kinds of the following characteristics than a lipid nanoparticle comprising other types of ionizable lipids:

(1) encapsulating a drug into the lipid nanoparticle with high efficiency;
(2) uniform size of the prepared lipid nanoparticle (or having a low PDI value); and/or
(3) excellent drug delivery efficiency to cancer tissue, and/or cancer cells;
(4) capability of long circulation in vivo; and/or
(5) excellent cancer prevention and/or treatment effects.

According to one embodiment, the lipid nanoparticle may have a pKa of 5 to 8, 5.5 to 7.5, 6 to 7, or 6.5 to 7. The pKa is an acid dissociation constant, and refers to what is generally used as an index indicating the acid strength of a target substance. The pKa value of the lipid nanoparticle is important in terms of in vivo stability of the lipid nanoparticle and drug release of the lipid nanoparticle. In one embodiment, the lipid nanoparticle showing the pKa value in the above range may be safely delivered to cancer tissue and/or cancer cells in vivo, and reach to the cancer tissue and/or cancer cells, and after endocytosis, exhibit a positive charge to release an encapsulated drug (the first anticancer agent) through electrostatic interaction with an anionic protein of the endosome membrane.

The phospholipid of the elements of the lipid nanoparticle according to one embodiment plays a role of covering and protecting a core formed by interaction of the ionizable lipid and drug (the first anticancer agent) in the lipid nanoparticle, and may facilitate cell membrane permeation and endosomal escape during intracellular delivery of the drug by binding to the phospholipid bilayer of a target cell.

For the phospholipid, a phospholipid which can promote fusion of the lipid nanoparticle according to one embodiment may be used without limitation, and for example, it may be one or more kinds selected from the group consisting of dioleoylphosphatidylethanolamine (DOPE), distearoylphosphatidylcholine (DSPC), palmitoyloleoylphosphatidylcholine (POPC), egg phosphatidylcholine (EPC), dioleoylphosphatidylcholine (DOPC), dipalmitoylphosphatidylcholine (DPPC), dioleoylphosphatidylglycerol (DOPG), dipalmitoylphosphatidylglycerol (DPPG), distearoylphosphatidylethanolamine (DSPE), phosphatidylethanolamine (PE), dipalmitoylphosphatidylethanolamine, 1,2-dioleoyl-sn-glycero-3-phosphoethanolamine, 1-palmitoyl-2-oleoyl-sn-glycero-3-phosphoethanolamine (POPE), 1-palmitoyl-2-oleoyl-sn-glycero-3-phosphocholine (POPC), 1,2-dioleoyl-sn-glycero-3-[phospho-L-serine] (DOPS), 1,2-dioleoyl-sn-glycero-3-[phospho-L-serine] and the like. In one embodiment, the lipid nanoparticle comprising DOPE may be effective in mRNA delivery (excellent drug delivery efficiency to mRNA), and in other embodiment, the lipid nanoparticle comprising DSPE

may be effective in siRNA delivery (excellent drug delivery efficiency to siRNA).

The cholesterol may provide morphological rigidity to lipid filling in the lipid nanoparticle and be dispersed in the core and surface of the nanoparticle to improve the stability of the nanoparticle.

Herein, "lipid-PEG (polyethyleneglycol) conjugate", "lipid-PEG", "PEG-lipid", "PEG-lipid", or "lipid-PEG" refers to a form in which lipid and PEG are conjugated, and means a lipid in which a polyethylene glycol (PEG) polymer which is a hydrophilic polymer is bound to one end. The lipid-PEG conjugate contributes to the particle stability in serum of the nanoparticle within the lipid nanoparticle, and plays a role of preventing aggregation between nanoparticles. In addition, the lipid-PEG conjugate may protect nucleic acids from degrading enzyme during in vivo delivery of the nucleic acids and enhance the stability of nucleic acids in vivo and increase the half-life of the drug encapsulated in the nanoparticle.

In the lipid-PEG conjugate, the PEG may be directly conjugated to the lipid or linked to the lipid via a linker moiety. Any linker moiety suitable for binding PEG to the lipid may be used, and for example, includes an ester-free linker moiety and an ester-containing linker moiety. The ester-free linker moiety includes not only amido (-C(O)NH-), amino (-NR-), carbonyl (-C(O)-), carbamate (-NHC(O)O-), urea (-NHC(O)NH-), disulfide (-S-S-), ether (-O-), succinyl (-(O)CCH2CH2C(O)-), succinamidyl (-NHC(O)CH2CH2C(O)NH-), ether, disulfide but also combinations thereof (for example, a linker containing both a carbamate linker moiety and an amido linker moiety), but not limited thereto. The ester-containing linker moiety includes for example, carbonate (-OC(O)O-), succinoyl, phosphate ester (-O-(O)POH-O-), sulfonate ester, and combinations thereof, but not limited thereto.

In one embodiment, the average molecular weight of the lipid-PEG conjugate may be 100 to 10000 daltons, 200 to 10000 daltons, 500 to 10000 daltons, 1000 to 10000 daltons, 1500 to 10000 daltons, 2000 to 10000 daltons, 100 to 7500 daltons, 200 to 7500 daltons, 500 to 7500 daltons, 1000 to 7500 daltons, 1500 to 7500 daltons, 2000 to 7500 daltons, 100 to 5000 daltons, 200 to 5000 daltons, 500 to 5000 daltons, 1000 to 5000 daltons, 1500 to 5000 daltons, 2000 to 5000 daltons, 100 to 3000 daltons, 200 to 3000 daltons, 500 to 3000 daltons, 1000 to 3000 daltons, 1500 to 3000 daltons, 2000 to 3000 daltons, 100 to 2600 daltons, 200 to 2600 daltons, 500 to 2600 daltons, 1000 to 2600 daltons, 1500 to 2600 daltons, 2000 to 2600 daltons, 100 to 2500 daltons, 200 to 2500 daltons, 500 to 2500 daltons, 1000 to 2500 daltons, 1500 to 2500 daltons, or 2000 to 2500 daltons.

For the lipid in the lipid-PEG conjugate, any lipid capable of binding to polyethyleneglycol may be used without limitation, and the phospholipid and/or cholesterol which are other elements of the lipid nanoparticle may be also used. Specifically, the lipid in the lipid-PEG conjugate may be ceramide, dimyristoylglycerol (DMG), succinoyl-diacylglycerol (s-DAG), distearoylphosphatidylcholine (DSPC), distearoylphosphatidylethanolamine (DSPE), or cholesterol.

In one embodiment, the lipid-PEG conjugate may be PEG bound to dialkyloxypropyl (PEG-DAA), PEG bound to diacylglycerol (PEG-DAG), PEG bound to a phospholipid such as phosphatidylethanolamine (PEG-PE), PEG conjugated to ceramide (PEG-CER, ceramide-PEG conjugate, ceramide-PEG, PEG-ceramide conjugate or PEG-ceramide), cholesterol or PEG conjugated to derivative thereof, PEG-c-DOMG, PEG-DMG, PEG-DLPE, PEG-DMPE, PEG-DPPC, PEG-DSPE(DSPE-PEG), and a mixture thereof, and for example, may be C16-PEG2000 ceramide, DMG-PEG 2000, 14:0 PEG2000 PE.

According to one embodiment, the case of comprising a lipid nanoparticle comprising a ceramide-PEG conjugate may have one or more kinds of the following characteristics than the case of comprising a lipid nanoparticle comprising other types of lipid-PEG conjugates:

(1) encapsulating a drug into the lipid nanoparticle with high efficiency;
(2) uniform size of the prepared lipid nanoparticle (or having a low PDI value); and/or
(3) excellent drug delivery efficiency to cancer tissue, and/or cancer cells;
(4) capability of long circulation in vivo; and/or
(5) excellent cancer prevention and/or treatment effects.

The PEG in the lipid-PEG conjugate is a hydrophilic polymer and has an ability to inhibit adsorption of serum proteins, and increases the circulation time of lipid nanoparticles in the body and can play a role of preventing aggregation between nanoparticles. In addition, the lipid-PEG conjugate may exhibit a stealth function in vivo to prevent degradation of nanoparticles.

The PEG may be what a functional group binds to a side not bound to a lipid (functionalized PEG). In this case, the functional group that can be used may be one or more kinds selected from the group consisting of succinyl group, carboxylic acid, maleimide, amine group, biotin, cyanur group and folate, and the like.

According to one embodiment, the lipid-PEG conjugate may be comprised in the lipid nanoparticle in an amount of 0.1 to 20 mol%, 0.25 to 20 mol%, 0.5 to 20 mol%, 1 to 20 mol%, 1.5 to 20 mol%, 2 to 20 mol%, 2.5 to 20 mol%, 3 to 20 mol%, 0.1 to 15 mol%, 0.25 to 15 mol%, 0.5 to 15 mol%, 1 to 15 mol%, 1.5 to 15 mol%, 2 to 15 mol%, 2.5 to 15 mol%, 3 to 15 mol%, 0.1 to 12.5 mol%, 0.25 to 12.5 mol%, 0.5 to 12.5 mol%, 1 to 12.5 mol%, 1.5 to 12.5 mol%, 2 to 12.5 mol%, 2.5 to 12.5 mol%, 3 to 12.5 mol%, 0.1 to 10 mol%, 0.25 to 10 mol%, 0.5 to 10 mol%, 1 to 10 mol%, 1.5 to 10 mol%, 2 to 10 mol%, 2.5 to 10 mol%, 3 to 10 mol%, 0.1 to 7.5 mol%, 0.25 to 7.5 mol%, 0.5 to 7.5 mol%, 1 to 7.5

mol%, 1.5 to 7.5 mol%, 2 to 7.5 mol%, 2.5 to 7.5 mol%, 3 to 7.5 mol%, 0.1 to 5 mol%, 0.25 to 5 mol%, 0.5 to 5 mol%, 1 to 5 mol%, 1.5 to 5 mol%, 2 to 5 mol%, 2.5 to 5 mol%, or 3 to 5 mol%.

In one embodiment, the drug delivery effect into cancer tissue and/or cancer cells of the lipid nanoparticle may be dependent on the content of the lipid-PEG conjugate comprised in the lipid nanoparticle.

For example, the lipid nanoparticle comprising the lipid-PEG conjugate in an amount of 0.1 to 20 mol%, 0.25 to 20 mol%, 0.5 to 20 mol%, 1 to 20 mol%, 1.5 to 20 mol%, 2 to 20 mol%, 2.5 to 20 mol%, 3 to 20 mol%, 0.1 to 15 mol%, 0.25 to 15 mol%, 0.5 to 15 mol%, 1 to 15 mol%, 1.5 to 15 mol%, 2 to 15 mol%, 2.5 to 15 mol%, 3 to 15 mol%, 0.1 to 12.5 mol%, 0.25 to 12.5 mol%, 0.5 to 12.5 mol%, 1 to 12.5 mol%, 1.5 to 12.5 mol%, 2 to 12.5 mol%, 2.5 to 12.5 mol%, 3 to 12.5 mol%, 0.1 to 10 mol%, 0.25 to 10 mol%, 0.5 to 10 mol%, 1 to 10 mol%, 1.5 to 10 mol%, 2 to 10 mol%, 2.5 to 10 mol%, 3 to 10 mol%, 0.1 to 7.5 mol%, 0.25 to 7.5 mol%, 0.5 to 7.5 mol%, 1 to 7.5 mol%, 1.5 to 7.5 mol%, 2 to 7.5 mol%, 2.5 to 7.5 mol%, 3 to 7.5 mol%, 0.1 to 5 mol%, 0.25 to 5 mol%, 0.5 to 5 mol%, 1 to 5 mol%, 1.5 to 5 mol%, 2 to 5 mol%, 2.5 to 5 mol%, or 3 to 5 mol% may have one or more kinds of the following characteristics than a lipid nanoparticle comprising a lipid-PEG conjugate in a content outside the above range:

(1) encapsulating the first anticancer agent with high efficiency;
(2) uniform size of the prepared lipid nanoparticle (or having a low PDI value); and/or
(3) excellent drug delivery efficiency to cancer tissue, and/or cancer cells;
(4) capability of long circulation in vivo; and/or
(5) excellent cancer prevention and/or treatment effects.

According to one embodiment, the cholesterol may be comprised in the lipid nanoparticle in an amount of 10 to 60 mol%, 20 to 60 mol%, 30 to 60 mol%, 34.5 to 60 mol%, 35 to 60 mol%, 39.5 to 60 mol%, 40 to 60 mol%, 43 to 60 mol%, 44 to 60 mol%, 10 to 55 mol%, 20 to 55 mol%, 30 to 55 mol%, 34.5 to 55 mol%, 35 to 55 mol%, 39.5 to 55 mol%, 40 to 55 mol%, 43 to 55 mol%, 44 to 55 mol%, 10 to 52.5 mol%, 20 to 52.5 mol%, 30 to 52.5 mol%, 34.5 to 52.5 mol%, 35 to 52.5 mol%, 39.5 to 52.5 mol%, 40 to 52.5 mol%, 43 to 52.5 mol%, 44 to 52.5 mol%, 10 to 51 mol%, 20 to 51 mol%, 30 to 51 mol%, 34.5 to 51 mol%, 35 to 51 mol%, 39.5 to 51 mol%, 40 to 51 mol%, 43 to 51 mol%, 44 to 51 mol%, 10 to 50 mol%, 20 to 50 mol%, 30 to 50 mol%, 34.5 to 50 mol%, 35 to 50 mol%, 39.5 to 50 mol%, 40 to 50 mol%, 43 to 50 mol%, 44 to 50 mol%, 10 to 45 mol%, 20 to 45 mol%, 30 to 45 mol%, 34.5 to 45 mol%, 35 to 45 mol%, 39.5 to 45 mol%, 40 to 45 mol%, 43 to 45 mol%, 44 to 45 mol%, 10 to 44.25 mol%, 20 to 44.25 mol%, 30 to 44.25 mol%, 34.5 to 44.25 mol%, 35 to 44.25 mol%, 39.5 to 44.25 mol%, 40 to 44.25 mol%, 43 to 44.25 mol%, 44 to 44.25 mol%, 10 to 44 mol%, 20 to 44 mol%, 30 to 44 mol%, 34.5 to 44 mol%, 35 to 44 mol%, 39.5 to 44 mol%, 40 to 44 mol%, 43 to 44 mol%, 44 to 44 mol%, 10 to 43 mol%, 20 to 43 mol%, 30 to 43 mol%, 34.5 to 43 mol%, 35 to 43 mol%, 39.5 to 43 mol%, or40 to 43 mol%.

According to one embodiment, the sum of the lipid-PEG conjugate and cholesterol may be comprised in the lipid nanoparticle in an amount of 40 to 60 mol%, 40 to 55 mol%, 40 to 53.5 mol%, 40 to 50 mol%, 40 to 47.5 mol%, 40 to 45 mol%, 40 to 44,5 mol%, 42 to 60 mol%, 42 to 55 mol%, 42 to 53.5 mol%, 42 to 50 mol%, 42 to 47.5 mol%, 42 to 45 mol%, 42 to 44,5 mol%, 44 to 60 mol%, 44 to 55 mol%, 44 to 53.5 mol%, 44 to 50 mol%, 44 to 47.5 mol%, 44 to 45 mol%, 44 to 44.5 mol%, 44.5 to 60 mol%, 44.5 to 55 mol%, 44.5 to 53.5 mol%, 44.5 to 50 mol%, 44.5 to 47.5 mol%, or 44.5 to 45 mol%.

According to one embodiment, the ionizable lipid may be comprised in the lipid nanoparticle in an amount of 10 to 60 mol%, 10 to 55 mol%, 10 to 50 mol%, 10 to 45 mol%, 10 to 42.5 mol%, 10 to 40 mol%, 10 to 35 mol%, 10 to 30 mol%, 10 to 26.5 mol%, 10 to 25 mol%, 10 to 20 mol%, 15 to 60 mol%, 15 to 55 mol%, 15 to 50 mol%, 15 to 45 mol%, 15 to 42.5 mol%, 15 to 40 mol%, 15 to 35 mol%, 15 to 30 mol%, 15 to 26.5 mol%, 15 to 25 mol%, 15 to 20 mol%, 20 to 60 mol%, 20 to 55 mol%, 20 to 50 mol%, 20 to 45 mol%, 20 to 42.5 mol%, 20 to 40 mol%, 20 to 35 mol%, 20 to 30 mol%, 20 to 26.5 mol%, 20 to 25 mol%, 25 to 60 mol%, 25 to 55 mol%, 25 to 50 mol%, 25 to 45 mol%, 25 to 42.5 mol%, 25 to 40 mol%, 25 to 35 mol%, 25 to 30 mol%, 25 to 26.5 mol%, 26.5 to 60 mol%, 26.5 to 55 mol%, 26.5 to 50 mol%, 26.5 to 45 mol%, 26.5 to 42.5 mol%, 26.5 to 40 mol%, 26.5 to 35 mol%, 26.5 to 30 mol%, 30 to 60 mol%, 30 to 55 mol%, 30 to 50 mol%, 30 to 45 mol%, 30 to 42.5 mol%, 30 to 40 mol%, 30 to 35 mol%, 35 to 60 mol%, 35 to 55 mol%, 35 to 50 mol%, 35 to 45 mol%, 35 to 42.5 mol%, 35 to 40 mol%, 40 to 60 mol%, 40 to 55 mol%, 40 to 50 mol%, 40 to 45 mol%, 40 to 42.5 mol%, 42.5 to 60 mol%, 42.5 to 55 mol%, 42.5 to 50 mol%, or 42.5 to 45 mol%.

According to one embodiment, the phospholipid may be comprised in the lipid nanoparticle in an amount of 5 to 30 mol%, 5 to 25 mol%, 5 to 20 mol%, 5 to 15 mol%, 5 to 13 mol%, 5 to 10 mol%, 10 to 30 mol%, 10 to 25 mol%, 10 to 20 mol%, 10 to 15 mol%, 10 to 13 mol%, 15 to 30 mol%, 15 to 25 mol%, 15 to 20 mol%, 20 to 30 mol%, or 20 to 25 mol%.

Herein, "mol% (mol%, mole percentage)" is expressed as a percentage by dividing the number of moles of a specific component by the sum of moles of all components and then multiplying by 100, and expressed as a formula, for example, it may be as Equation 1 below.

(Equation 1)

$$mol\% = (\text{moles of a specific component}) / (\text{sum of moles of all components}) \times 100$$

The lipid nanoparticle may comprise the ionizable lipid : phospholipid : cholesterol : lipid-PEG conjugate at a molar ratio of 20 to 50 : 10 to 30 : 10 to 60 : 0.25 to 10, at a molar ratio of 20 to 50 : 10 to 30 : 20 to 60 : 0.25 to 10, at a molar ratio of 20 to 50 : 10 to 30 : 30 to 60 : 0.5 to 5, at a molar ratio of 25 to 45 : 10 to 25 : 40 to 50 : 0.5 to 3, at a molar ratio of 25 to 45 : 10 to 20 : 40 to 55 : 0.5 to 3, at a molar ratio of 25 to 45 : 10 to 20 : 40 to 55 : 1.0 to 1.5, at a molar ratio of 40 to 45 : 10 to 15 : 40 to 45 : 0.5 to 3.0, at a molar ratio of 40 to 45 : 10 to 15 : 40 to 45 : 0.5 to 3, at a molar ratio of 40 to 45 : 10 to 15 : 40 to 45 : 1 to 1.5, at a molar ratio of 25 to 30: 17 to 22; 50 to 55 : 0.5 to 3.0, at a molar ratio of 25 to 30: 17 to 22; 50 to 55 : 1.0 to 2.5, at a molar ratio of 25 to 30: 17 to 22; 50 to 55 : 1.5 to 2.5. while maintaining the sum of the moles of the lipid-PEG conjugate and cholesterol constant, among the components comprised in the lipid nanoparticle, the moles of cholesterol are decreased as much as the number of moles of the lipid-PEG conjugate is increased, and thereby the molar ratio of the components can be maintained.

Herein, the molar ratio means a ratio of moles.

The lipid nanoparticle may comprise the ionizable lipid of 20 to 50 parts by weight, phospholipid of 10 to 30 parts by weight, cholesterol of 30 to 60 parts by weight, and lipid-PEG conjugate of 0.1 to 10 parts by weight.

Herein, "parts by weight" means a weight ratio of each component comprised.

The lipid nanoparticle may comprise the ionizable lipid of 20 to 50 parts by weight, phospholipid of 10 to 30 parts by weight, cholesterol of 40 to 60 parts by weight, and lipid-PEG conjugate of 1.5 to 3 parts by weight based on the total nanoparticle weight. Specifically, the lipid nanoparticle may comprise the ionizable lipid of 25 to 50 parts by weight, phospholipid of 10 to 20 parts by weight, cholesterol of 35 to 55 parts by weight, and lipid-PEG conjugate of 0.5 to 5.0 parts by weight based on the total nanoparticle weight.

The pharmaceutical composition comprising a lipid nanoparticle comprising the ionizable lipid, cholesterol, phospholipid, and/or lipid-PEG conjugate in the above range (range of molar ratio, part by weight, and/or % by weight) may have excellent (i) stability of the lipid nanoparticle; (ii) encapsulation efficiency of the anticancer agent; (iii) drug delivery efficiency into cancer tissue and/or cancer cells; (iv) long circulation effect in vivo; and/or (iv) cancer prevention and/or treatment effects, than the case of comprising the lipid nanoparticle comprising the ionizable lipid, cholesterol, phospholipid and/or lipid-PEG conjugate outside the above range.

Herein, "targeting" and "localization" to the cancer tissue, and/or cancer cells of the lipid nanoparticle may be internalization in the tissue or cells, and may mean internalization inside the nucleus as it can penetrate the nuclear membrane.

The pharmaceutical composition may be one in which the first anticancer agent (for example, an anionic drug and/or a physiologically active substance such as nucleic acid) is encapsulated, and the first anticancer agent is stably encapsulated in the lipid nanoparticle with high efficiency, thereby exhibiting excellent cancer prevention and/or treatment effects by the pharmaceutical composition according to one example.

In one embodiment, the weight ratio of the ionizable lipid and the first anticancer agent (for example, an anionic drug, a nucleic acid, or a combination thereof) comprised in the lipid nanoparticle may be 1 to 20 : 1, 1 to 15 : 1, 1 to 10 : 1, 5 to 20 : 1, 5 to 15 : 1, 5 to 10 : 1, 7.5 to 20 : 1, 7.5 to 15 : 1, or 7.5 to 10 : 1.

When the composition according to one embodiment comprises (1) the ionizable lipid; and (2) the first anticancer agent (for example, an anionic drug, a nucleic acid, or a combination thereof) at a weight ratio in the above range, the encapsulation ratio of the first anticancer agent (for example, an anionic drug, a nucleic acid, or a combination thereof) inside the lipid nanoparticle; (ii) anticancer agent delivery efficiency into cancer tissue and/or cancer cells; (iii) long circulation effect in vivo; and/or (iv) cancer prevention and/or treatment effects may be higher (excellent) than a composition comprising a lipid nanoparticle comprising (1) the ionizable lipid; and (2) the first anticancer agent at a weight ratio outside the above range.

The lipid nanoparticle in which the first anticancer agent is encapsulated may have an average diameter of 20nm to 200nm, 20 to 180nm, 20nm to 170nm, 20nm to 150nm, 20nm to 120nm, 20nm to 100nm, 20nm to 90nm, 30nm to 200nm, 30 to 180nm, 30nm to 170nm, 30nm to 150nm, 30nm to 120nm, 30nm to 100nm, 30nm to 90nm, 40nm to 200nm, 40 to 180nm, 40nm to 170nm, 40nm to 150nm, 40nm to 120nm, 40nm to 100nm, 40nm to 90nm, 50nm to 200nm, 50 to 180nm, 50nm to 170nm, 50nm to 150nm, 50nm to 120nm, 50nm to 100nm, 50nm to 90nm, 60nm to 200nm, 60 to 180nm, 60nm to 170nm, 60nm to 150nm, 60nm to 120nm, 60nm to 100nm, 60nm to 90nm, 70nm to 200nm, 70 to 180nm, 70nm to 170nm, 70nm to 150nm, 70nm to 120nm, 70nm to 100nm, 70nm to 90nm, 80nm to 200nm, 80 to 180nm, 80nm to 170nm, 80nm to 150nm, 80nm to 120nm, 80nm to 100nm, 80nm to 90nm, 90nm to 200nm, 90 to 180nm, 90nm to 170nm, 90nm to 150nm, 90nm to 120nm, or 90nm to 100nm, for easy introduction into cancer tissue and/or cancer cells. As the nanoparticle having a size of the diameter within the above range can remain in blood for a long time, an opportunity of reach the target tumor tissue can be increased. The lipid nanoparticle having

a diameter size within the above range may exhibit an EPR (Enhanced Permeability and Retention). The EPR effect means that molecules having a specific size tend to accumulate in tumor tissue than normal tissue. The lipid nanoparticle having a diameter size within the range is less excreted to kidney than a lipid nanoparticle having a diameter size outside the range, and the activity of immunocytes is not induced, so that delivery into cacner cells and/or cance tissue can be effectively achieved.

In one embodiment, the composition comprising the lipid nanoparticle having a diameter within the above range has (i) anticancer agent delivery efficiency into cancer tissue and/or cancer cells; (ii) an increase of enhanced permeability and retention (EPR effect) into cancer tissue and/or cancer cells; and/or (iii) cancer prevention and/or treatment effects, excellent than a composition comprising a lipid nanoparticle having a diameter exceeding the upper limit in the above range. The lipid nanoparticle according to one embodiment exhibits a positive charge under the acidic pH condition by showing a pKa of 5 to 8, 5.5 to 7.5, 6 to 7, or 6.5 to 7, and may encapsulate a drug with high efficiency by easily forming a complex with a drug through electrostatic interaction with a therapeutic agent such as the first anticancer agent (for example, a nucleic acid and an anionic drug (for example, protein)) showing a negative charge, and it may be usefully used as a composition for preventing and/or treating cancer comprising the first anticancer agent (for example, nucleic acid).

Herein, "encapsulation" refers to encapsulating a delivery substance for surrounding and embedding it in vivo efficiently, and the first anticancer agent (drug) encapsulation rate (encapsulation efficiency) means the content of the first anticancer agent (drug) encapsulated in the lipid nanoparticle for the total drug content used for preparation.

In the composition according to one embodiment, the encapsulation efficiency of the first anticancer agent may be 70% or more, 75% or more, 80% or more, 85% or more, 90% or more, 91% or more, 92% or more, 94% or more, over 80% to 99% or less, over 80% to 97% or less, over 80% to 95% or less, 85% or more to 95% or less, 87% or more to 95% or less, 90% or more to 95% or less, 91% or more to 95% or less, 91% or more to 94% or less, over 91% to 95% or less, 92% or more to 99% or less, 92% or more to 97% or less, or 92% or more to 95% or less.

According to one embodiment, the encapsulation efficiency may be calculated by commonly used methods, and for example, the first anticancer agent (drug) encapsulation efficiency may be calculated by the following Equation 2, by treating Triton-X to the lipid nanoparticle according to one embodiment and measuring the fluorescence intensity of the Triton-X-treated and Triton-X-untreated lipid nanoparticles in a specific wavelength bandwidth (for example, excitation: 480 ~ 490nm, emission: 520 ~ 530nm).

(Equation 2)

Drug encapsulation efficiency (%) = (Fluorescence intensity (fluorescence) of the Triton-X-treated lipid nanoparticle - Fluorescence intensity (fluorescence) of the Triton-X-untreated lipid nanoparticle)/(Fluorescence intensity (fluorescence) of the Triton-X-treated lipid nanoparticle) X 100

The first anticancer agnet may be an anionic drug, a nucleic acid, or a combination thereof exhibiting cancer treatment and/or prevention effects.

The nucleic acid may be one or more kinds selected from the group consisting of small interfering RNA (siRNA), ribosome ribonucleic acid (rRNA), ribonucleic acid (RNA), deoxyribonucleic acid (DNA), complementary deoxyribonucleic acid (cDNA), aptamer, messenger ribonucleic acid (mRNA), transfer ribonucleic acid (tRNA), antisense oligonucleotide, shRNA, miRNA, ribozyme, PNA, DNAzyme and sgRNA for gene editing, and the like, but not limited thereto.

Herein, the term "siRNA" refers to double stranded RNA (duplex RNA) which can induce RNAi (RNA interference) through cleavage of specific mRNA, or single stranded RNA that has a double stranded form inside the single stranded RNA. It consists of a sense RNA strand having the sequence homologous to mRNA of a target gene and an antisense RNA strand having the sequence complementary thereto. As siRNA can inhibit expression of a target gene, it is provided by an effective gene knock-down method or method of gene therapy. Binding between double strands is carried out through hydrogen bonding between nucleotides, and not all nucleotides within the double strand must be complementary and completely bound.

The length of siRNA may be about 15 to 60, specifically about 15 to 50, about 15 to 40, about 15 to 30, about 15 to 25, about 16 to 25, about 19 to 25, about 20 to 25, or about 20 to 23 nucleotides. The siRNA length means the number of nucleotides on one side of the double stranded RNA, that is, the number of base pairs, and in case of single

stranded RNA, means the length of the double strand inside the single stranded RNA. In addition, siRNA may be composed of nucleotides introduced with various functional groups for the purpose of increasing blood stability or weakening an immune response, and the like.

Herein, the term "antisense oligonucleotide" may be modified at a position of one or more bases, sugars or backbones to enhance the efficacy (De Mesmaeker et al., Curr Opin Struct Biol., 5(3):343-55, 1995). The oligonucleotide backbone may be modified by phosphorothioate, phosphotriester, methyl phosphonate, short-chain alkyl, cycloalkyl, short-chain heteroatomic, heterocyclic intersaccharide binding, and the like. In addition, the antisense oligonucleotide may comprise one or more substituted sugar moieties. The antisense oligonucleotide may comprise a modified base. The modified base includes hypoxanthine, 6-methyladenine, 5-me pyrimidine (particularly, 5-methylcytosine), 5-hydroxymethylcytosine (HMC), glycosyl HMC, gentiobiosyl HMC, 2-aminoadenne, 2-thiouracil, 2-thiothymine, 5-bromouracil, 5-hydroxymethyluracil, 8-azaguanine, 7-deazaguanine, N6(6-aminohexyl)adenine, 2,6-diaminopurine, and the like.

Herein, "single stranded deoxyribonucleic acid (ssDNA)" means a single stranded oligonucleotide which binds to specific target DNA selectively and induces an antigene effect.

Herein, "aptamer" means an oligonucleotide (generally, 20 ~ 80 nt DNA or RNA) which binds to a specific target. Preferably, herein, "aptamer" means an oligonucleotide aptamer (e.g., DNA or RNA aptamer).

Herein, "mRNA" means synthetic mRNA (in vitro transcribed mRNA) capable of expressing a gene.

Herein, "shRNA" means single-stranded 50 to 70 nucleotides, and forms a stem-loop (stemloop) structure in vivo. On both sides of the loop of 5 to 10 nucleotides, complementarily, long RNA of 19 to 29 nucleotides are base-paired to form a double-stranded stem.

Herein, "miRNA (microRNA)" means a single stranded RNA molecule which controls gene expression and consists of full length 21 to 23 nucleotides. miRNA is an oligonucleotide which is not expressed in a cell, and has a short stem-loop structure. miRNA has full or partial homology with one or two or more mRNA (messenger RNA) and suppresses target gene expression through complementary binding to the mRNA.

Herein, "ribozyme" is a kind of RNA, and is RNA which recognizes a nucleotide sequence of specific RNA and has the same function as enzyme cutting it by itself. The ribozyme is a complementary nucleotide sequence of a target messenger RNA strand and consists of a region that binds with specificity and a region that cuts the target RNA.

Herein, "DNAzyme" is a single stranded DNA molecule having enzyme activity, and DNAzyme consisting of 10 to 23 nucleotides (10-23 DNAzyme) cuts an RNA strand at a specific position under the physiologically similar condition. The 10-23 DNAzyme cuts between any exposed purines and pyrimidines without base pairing. The 10-23 DNAzyme consists of an active site (catalytic domain) of enzyme consisting of 15 conserved nucleotide sequences (for example, 5'-GGCTAGCTACAACGA-3') and an RNA substrate binding domain consisting of 7 ~ 8 DNA nucleotide sequences which recognize RNA substrates to the left and right of the active domain of the enzyme afore-described.

Herein, "PNA (Peptide nucleic acid)" is a molecule having all properties of nucleic acids and proteins, and means a molecule capable of complementarily binding to DNA or RNA. The PNA was first reported in 1999 as similar DNA in which nucleobases are linked by peptide bonds (document [Nielsen PE, Egholm M, Berg RH, Buchardt O, "Sequence-selective recognition of DNA by strand displacement with a thymine-substituted polyamide", Science 1991, Vol. 254: pp1497-1500]). The PNA is not found in the natural world and is artificially synthesized by a chemical method. The PNA causes a hybridization reaction with a natural nucleic acid of a complementary nucleotide sequence to form a double strand. PNA/DNA double strands are more stable than DNA/DNA double strands for the same length. As a backbone of peptides, N-(2-aminoethyl)glycine repeatedly linked by amide bonds is most commonly used, and in this case, the backbone of the peptide nucleic acid is electrically neutral different from the backbone of the natural nucleic acid. 4 nucleotides present in PNA have almost the same spatial size and distance between nucleotides as in case of the natural nucleic acid. The PNA is not only chemically more stable than the natural nucleic acid, but also biologically stable because it is not degraded by nuclease or protease.

Herein, "sgRNA" is an oligonucleotide (generally, RNA molecule) binding to a specific DNA target, and means a complex single RNA molecule of crispr RNA (crRNA) and tracer (tracrRNA). It is an RNA molecule which is used for recognizing a specific DNA sequence with Cas9 nuclease in the CRISPR system and enables selective gene cleavage, and approximately, comprises a 20-nt sequence capable of complementarily binding to DNA, and has a total length of 100 nt.

Herein, "gene editing protein" refers to Cas9, spCas9, cjCas9, casX, CasY and Cpf1, and the like, and refers to a protein that recognizes a target DNA nucleotide sequence with sgRNA to cause DNA cleavage.

The composition for drug delivery according to one embodiment may comprise Cas9 mRNA with high encapsulation efficiency. The previously known composition for delivering Cas9 mRNA has a limitation in using it as a composition for delivering Cas9 mRNA, as it comprises Cas9 mRNA at a low ratio. On the other hand, the lipid nanoparticle according to one embodiment may comprise Cas9 mRNA with high encapsulation efficiency, specifically, encapsulation efficiency of 70% or more, and thus it may be usefully utilized for gene editing therapy.

The anionic drug may be an anionic biopolymer-drug conjugate such as various kinds of peptides, protein drugs, protein-nucleic acid structures or hyaluronic acid-peptide conjugates, hyaluronic acid-protein conjugates, which

have an anion, and the like. The non-restrictive examples of the protein drugs may be apoptosis-inducing factors (e.g., cytocrome C, caspase 3/7/8/9, etc.) and including gene editing proteins such as Cas 9, cpf1 which are gene editing scissors, and various intracellular proteins (e.g., transcription factors), and the like.

The pharmaceutical composition for preventing or treating cancer according to one embodiment may comprise a lipid nanoparticle in which an anionic drug and/or a nucleic acid is encapsulated inside.

The cancer may be caused by HPV (human papilloma virus) infection. For example, the cancer caused by HPV infection, is not limited thereof, but may be selected from the group consisting of cervical cancer, vagina cancer, vulvacancer, anal cancer, penis cancer, tonsil cancer, pharynx cancer, larynx cancer, head and neck cancer and lung adenocarcinoma.

The cancer may be selected from the group consisting of cervical cancer, vagina cancer, vulvacancer, anal cancer, penis cancer, tonsil cancer, pharynx cancer, larynx cancer, head and neck cancer, lung adenocarcinoma, non-small cell lung cancer, lung squamous cell carcinoma, lung cancer, stomach cancer, sarcoma, lymphoma, Hodgkin lymphoma, chronic or acute leukemia, thymic carcinoma, epithelial cancer, salvinary gland cancer, liver cancer, stomach cancer, thyroid cancer, parathyroid cancer, ovarian cancer, breast cancer, prostate cancer, esophageal cancer, pancreatic cancer, glioma, multiple myeloma, renal cell carcinoma, bladder cancer, choriocarcinoma, colon cancer, oral cancer, skin cancer, melanoma, bone cancer, rectal cancer, small intestine cancer, endocrine gland cancer, adrenal cancer, soft tissue sarcoma, urethral cancer, spinal cord tumor, brainstem glioma and pituitary adenoma.

The pharmaceutical composition may be administered by various routes including parenteral administration into mammals including humans, and parenteral administration may be applied intravenously, subcutaneously, intraperitoneally or locally, and the dosage varies depending on the condition and body weight of the patient, degree of disease, drug form, administration route and time, but may be appropriately selected by those skilled in the art. When the pharmaceutical composition according to one embodiment is formulated, it is prepared by using a diluent or excipient such as a filler, an extender, a binding agent, a wetting agent, a disintegrant, a surfactant, and the like used commonly.

The formulation for parenteral administration includes a sterilized aqueous solution, a non-aqueous solvent, a suspended solvent, emulsion, a lyophilized formulation, a suppository, and the like.

As the non-aqueous solvent and suspended solvent, propylene glycol, polyethylene glycol, plant oil such as olive oil, injectable ester such as ethyl oleate, and the like may be used. As a base compound of the suppository, witepsol, macrogol, tween 61, cacao butter, laurin butter, glycerol, gelatin, and the like.

The pharmaceutical composition according to one embodiment is administered in a pharmaceutically effective dose. Herein, "pharmaceutically effective dose" means an amount sufficient to treat disease at a reasonable benefit/risk ratio applicable to medical treatment, and the effective dose level may be determined depending on factors including the patient's disease type, severity, drug activity, drug sensitivity, administration time, administration route and excretion rate, treatment period and concomitant drugs, and other factors well known in the medical field. The pharmaceutical composition according to one embodiment may be administered as an individual therapeutic agent or may be administered in combination with other therapeutic agents, and may be administered sequentially or simultaneously with conventional therapeutic agents, and may be singly or multiply administered. It is important to administer an amount capable of obtaining the maximum effect with a minimum amount without side effects, in consideration of all of the above factors, and this may be easily determined by those skilled in the art.

Specifically, the effective dose of the compound according to the present invention may vary depending on the patient's age, gender and body weight, and may be administered daily or every other day, or administered by dividing into 1 to 3 times a day. However, it may be increased or reduced depending on the administration route, severity of obesity, gender, body weight, age, and the like, and therefore, the above dose does not limit the scope of the present invention in any way.

In one specific embodiment, the pharmaceutical composition may be administered in a dose of 0.1 to 100mg/kg, 0.1 to 50mg/kg, 1 to 10mg/kg, or 1 to 5mg/kg, based on the concentration of the drug (anionic drug, nucleic acid or combination thereof) comprised in the pharmaceutical composition.

Other aspect may provide a pharmaceutical composition for preventing cancer for use in radiation therapy in combination; a pharmaceutical composition for enhancing radiation sensitivity; or a pharmaceutical composition for treating cancer in combination with radiation therapy, comprising the above pharmaceutical composition. The pharmaceutical composition can further increase the effect of treating cancer by improving damage sensitivity of cancer cells to radiation during treatment of cancer, and/or can be used as a composition or agent to be treated so that death of cancer cells by radiation is easily induced by inhibiting resistance of cancer cells to radiation, and therefore, a composition for comprising the pharmaceutical composition can be used in combination with radiation therapy.

Herein, enhancing radiation sensitivity means enhancing sensitivity of cells to radiation in treatment of diseases using radiation. Through this, the radiation therapy efficiency can be increased, and in particular, when treated in parallel during cancer treatment, the radiation sensitivity of cancer cells is enhanced, os the killing effect and proliferation inhibiting effect of cancer cells can be exhibited.

The pharmaceutical composition for cancer treatment for use in radiation therapy in combination (or composition

for enhancing radiation sensitivity, pharmaceutical composition for cancer treatment for use in combination with radiation therapy) according to one embodiment is may comprise an expression or activity inhibitor ofE6 and/or E7 proteins of HPV 16 type (HPV16) or HPV 18 type (HPV18) virus; phosphomevalonate kinase (PMVK) protein; or a combination thereof, as the first anticancer agent. The PMVK expression inhibitor, E6, and/or E7 expression inhibitors may be selected from the group consisting of an antisense nucleotide, small interfering RNA (siRNA), and short hairpin RNA (shRNA) which complementarily bind to mRNA of the PMVK, E6, and/or E7 genes. The E6 and/or E7 activity inhibitors of the PMVK activity inhibitor and/or the HPV16 (or HPV 18 type) virus may be selected from the group consisting of a peptide, peptide mimetics, and an aptamer which specifically bind to E6 and/or E7 proteins of the PMVK, HPV16 (or HPV 18 type) virus.

In one embodiment, the pharmaceutical composition may be administered in combination with irradiation during treatment of cancer. The "irradiation" refers to a local treatment method which damages DNA of malignant cells. Normal cells have a greater ability to repair this damage than tumor cells. Irradiation means treatment using this difference, and includes a method of treatment using radiation meant commonly. Irradiation can be divided into radical (red) radiation therapy, adjuvant radiation therapy, and palliative radiation therapy according to the purpose of treatment. Radical (red) radiation therapy refers to radiation therapy for the purpose of complete cure, when a tumor is relatively limited to a local area, and no distant metastasis is present. Adjuvant radiation therapy refers to radiation therapy performed for the purpose of preventing local recurrence after a surgical operation. By using radiation therapy in combination, not only recurrence is simply reduced, but also the range of surgery is reduced to maintain function. Palliative radiation therapy is radiation therapy performed for the purpose of relieving symptoms caused by cancer.

Radiation is a treatment method that kills cancer cells using high-energy radiation, but affects normal tissue surrounding it as well as cancer cells, so side effects may occur due to treatment. Examples thereof include skin changes, hair loss, nausea and vomiting, diarrhea, mucositis/esophagitis, xerostomia, changes in reproductive function, and the like.

The pharmaceutical composition for cancer treatment for use in radiation theraphy in combination; pharmaceutical composition for enhancing radiation sensitivity; or pharmaceutical composition for cancer treatment for use in combination with radiation therapy according to one embodiment can reduce such side effects by reducing a requirement of radiation. In addition, in not only cancer cells sensitive to radiation, but also cancer cells resistant to radiation, radiation sensitivity can be increased.

The pharmaceutical composition for treating cancer for use in radiation therapy in combination (or pharmaceutical composition for treating cancer for use in combination with radiation therapy) comprising a lipid nanoparticle which comprises an ionizable lipid, cholesterol, a phospholipid, and/or a lipid-PEG conjugate in the afore-mentioned range (range of molar ratio, part by weight, and/or % by weight) may have excellent effects of killing cancer cells and treating cancer by increasing sensitivity of cancer cells, tumor cells and cancer tissue to radiation, than the case of comprising a lipid nanoparticle which comprises an ionizable lipid, cholesterol, a phospholipid, and/or a lipid-PEG conjugate outside the above range.

In one embodiment, according to one embodiment, the case of comprising a lipid nanoparticle which comprises 1,4-bis(3-aminopropyl)piperazine, and/or a ceramide-PEG conjugate may have excellent effects of killing cancer cells and treating cancer by increasing sensitivity of cancer cells, tumor cells and cancer tissue to radiation, than the case of comprising a lipid nanoparticle which comprises another type of lipid-PEG conjugate.

Other aspect may provide a pharmaceutical composition for use in combination with an anticancer agent; or a pharmaceutical composition for enhancing anticancer treatment, further comprising the pharmaceutical composition and a second anticancer agent. The pharmaceutical composition is as described above.

The second anticancer agent may be one or more kinds selected from the group consisting of gossypol, nitrogen mustard, imatinib, oxaliplatin, rituximab, erlotinib, neratinib, lapatinib, gefitinib, vandetanib, nilotinib, semaxanib, bosutinib, axitinib, cediranib, lestaurtinib, trastuzumab, gefitinib, bortezomib, sunitinib, carboplatin, bevacizumab, cisplatin, cetuximab, Viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracil, oteracil, azacitidine, methotrexate, uracil, cytarabine, fluorouracil, fludarabine, enocitabine, flutamide, decitabine, mercaptopurine, thioguanine, cladribine, carmofur, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, peplomycin, temsirolimus, temozolomide, busulfan, ifosfamide, cyclophosphamide, melphalan, altretamine, dacarbazine, thiotepa, nimustine, chlorambucil, mitolactol, leucovorin, tretinoin, exemestane, aminogluthetimide, anagrelide, navelbine, fadrozole, tamoxifen, toremifene, testolactone, anastrozole, letrozole, vorozole, bicalutamide, lomustine and carmustine.

In one embodiment, the second anticancer agent may be not encapsulated inside the lipid nanoparticle comprised in the pharmaceutical composition.

Other aspect may provide a method for enhancing sensitivity to chemotherapy or radiotherapy of cacner,

comprising administering the pharmaceutical composition in combination, during performing chemotherapy or radiotherapy for treatment of cancer (for example, cancer caused by HPV infection).

The "administering in combination" may perform administering the pharmaceutical composition first before several hours (for example, before 4 hours) before performing chemotheraphy, in case of chemotherapy. In addition, in case of radiotherapy, the pharmaceutical composition may be administered several times (for example, 2~4 times) over 2 days, after 1 day of irradiation.

Other aspect provides a method for prevention or treatment of cancer comprising administering the pharmaceutical composition into a patient. The cancer is as described above.

The method for prevention or treatment of cancer according to one embodiment may further comprise confirming (selecting) a patient in need of prevention and/or treatment of cancer. In one embodiment, the patient may be a patient who has undergone radiotherapy and/or chemotherapy; a patient undergoing radiotherapy and/or chemotherapy; and/or a patient scheduled to undergo radiotherapy and/or chemotherapy.

A target subject to which the method for treatment is applied means a mammal including mice, livestock, and the like, which includes humans, who have developed or may develop cancer, but not limited thereto. By administering the pharmaceutical composition (or pharmaceutical composition for use in combination with radiotherapy and/or an anticancer agent) comprising a lipid nanoparticle which comprises an ionizable lipid, cholesterol, a phospholipid, and/or lipid-PEG conjugate in the afore-mentioned range (range of molar ratio, part by weight, and/or % by weight) into the target subject, the subject can be efficiently treated.

Herein, "administration" means introducing the pharmaceutical composition into a target subject by any appropriate method, and as the administration route, it may be administered through various oral or parenteral (for example, intravenous, subcutaneous, or local application) routes as long as it can reach target tissue.

The method for prevention or treatment may comprise administering the pharmaceutical composition (or pharmaceutical composition for use in combination with radiotherapy and/or an anticancer agent) in a pharmaceutically effective dose. An appropriate total daily amount to be used may be determined by treatment within the scope of correct medical judgement, and it may be administered once or divided into several times. However, a specific therapeutically effective dose for a specific patient may be applied differently depending on various factors including the type and extent of reaction to be achieved, a specific composition including whether another agent is used in some cases, the patient's age, body weight, general health condition, gender and diet, administration time, administration route and secretion rate of the composition, treatment period, a drug which is used together or used simultaneously with a specific composition, and similar factors well known in the medical field.

Other aspect relates to a method for treatment for cancer comprising administering the pharmaceutical composition into an animal (for example, animal except for humans); and irradiating radiation.

When radiation is irradiated after administering the pharmaceutical composition, the radiotherapy effect, and further, cancer treatment effect may be significantly enhanced according to the synergistic effect.

The animal to be treated may be any mammal including humans, livestock, and pets, but not limited thereto.

For the irradiation, any irradiation method conventionally used for radiotherapy of cancer, or a radiation irradiation method for cancer developed later may be applied.

Other aspect may provide a composition for killing cancer cells comprising the lipid nanoparticle and the first anticancer agent. Other aspect may provide a method for killing cancer cells comprising encapsulating the first anticancer agent into the lipid nanoparticle. The cancer cells which may be killed by the composition according to one embodiment may mean the above-mentioned cancer cells.

[ADVANTAGEOUS EFFECTS]

The pharmaceutical composition according to one embodiment has excellent biocompatibility and can deliver a gene therapeutic agent with high efficiency, and thus it has an excellent effect of preventing or treating cancer, and has an excellent effect of preventing or treating cancer even when used in combination with an anticancer agent and/or radiation therapy.

[BRIEF DESCRIPTION OF THE DRAWINGS]

FIG. 1a shows an exemplary structure of the lipid nanoparticle according to one example, and FIG. 1b shows an image of observing the nanoparticle according to one example by Cryo-TEM.

FIG. 2 shows the result of 1H NMR (room temperature, 400MHz) of 246-C10 in $CDCl_3$.

FIG. 3a (241-C10 LNP to 243-C10 LNP) and FIG. 3b (244-C10 LNP to 246-C10 LNP) show the result of measuring the fluorescence intensity shown by each lipid nanoparticle in a solution having a range of pH 4.1 to pH 9.6.

FIG. 4a and FIG. 4b are results showing the intracellular gene delivery efficiency of each nanoparticle. Specifically, FIG. 4a shows the luminescence intensity measured by transforming LNP encapsulating mRNA (luc mRNA) encoding luciferase into HeLa cell and then dissolving the cell, and FIG. 4b shows the result of measuring luciferase expression after transforming LNP encapsulating siRNA to Hela-Luc cancer cell.

FIG. 5a to FIG. 5c are results showing gene delivery efficiency in a mouse of the nanoparticle. Specifically, FIG. 5a shows the result of confirming biodistribution in cancer tissue, liver and kidney by IVIS after 48 hours of encapsulating fluorescence-attached siGFP with LNP and the injecting it into a mouse model, and FIG. 5b shows the result of measuring the increase and decrease of luciferase expression with bioluminescence equipment by administering the lipid nanoparticle comprising siLuc to the Hela-Luc xenograft mouse model.

FIG. 5c shows the increase and decrease in luciferase expression according to the injection method after administering the lipid nanoparticle comprising siLuc to the Hela-Luc xenograft mouse model as a bioluminescence graph.

FIG. 6a shows an apoptosis effect by the nanoparticle comprising siRNA in a head and neck squamous cell cancer cell line, and a cervical cancer cell line.

FIG. 6b shows the increase and decrease of expression of mRNA genes by the nanoparticle comprising siRNA in a head and neck squamous cell cancer cell line, and a cervical cancer cell line.

FIG. 6c shows the increase and decrease of expression of proteins by the nanoparticle comprising siRNA in a head and neck squamous cell cancer cell line, and a cervical cancer cell line.

FIG. 7a shows an apoptosis effect by the nanoparticle comprising siRNA in a pancreatic cancer cell line, a lung cancer cell line, a breast cancer cell line and a cervical cancer cell line.

FIG. 7b shows the increase and decrease of mRNA gene expression by the nanoparticle comprising siRNA in a pancreatic cancer cell line, a lung cancer cell line, a breast cancer cell line and a cervical cancer cell line.

FIG. 7c shows the increase and decrease of protein expression by the nanoparticle comprising siRNA in a pancreatic cancer cell line, a lung cancer cell line, a breast cance cell line and a cervical cancer cell line.

FIG. 7d shows the apoptosis by the nanoparticle comprising siRNA and radiation in a pancreatic cancer cell line, a lung cancer cell line and a cervical cancer cell line.

FIG. 7e shows the increase and decrease of protein expression by the nanoparticle comprising siRNA and radiation in a pancreatic cancer cell line, a lung cancer cell line and a cervical cancer cell line.

FIG. 7f shows the increase and decrease of siRNA delivery and mRNA gene expression by the nanoparticle comprising siRNA against radiation in a mouse model transplanted with a pancreatic cancer cell line.

FIG. 7g shows the increase and decrease of siRNA delivery and mRNA gene expression by the nanoparticle comprising siRNA against radiation in a mouse model transplanted with a lung cancer cell line.

[MODE FOR INVENTION]

The present invention will be described in more detail by the following examples, but the scope is not intended to be limited by the following examples.

## Example 1. Preparation of ionizable lipids

### Example 1-1. Preparation of ionizable lipids

Ionizable lipids were synthesized by reacting the amine-based compounds of Table 1 below comprising a 6-membered heterocyclic tertiary amine and 1,2-epoxidodecane (hereinafter, C10) (Sigma-Aldrich, USA) at a molar ratio of 1:n (n = primary amine x 2 + secondary amine x 1).

[Table 1]

| Name | Chemical formula |
|---|---|
| **241** | 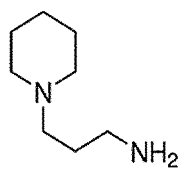 |

(continued)

| Name | Chemical formula |
|---|---|
| **242** | 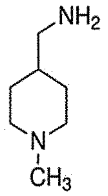 |
| **243** | 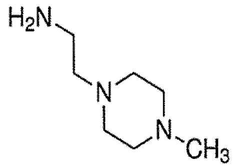 |
| **244** | 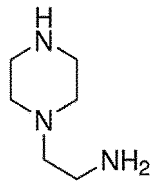 |
| **245** | 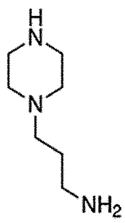 |
| **246** | 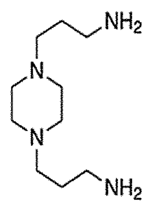 |

Specifically, each of 241 to 246 amines of the Table 1 and epoxide (C 10) were added at a molar ratio of 1:n (n = primary amine x 2 + secondary amine x 1) in a 5ml vial with a magnetic bar and reacted in a stirrer at 750rpm, 90°C for 3 days. Then, after purifying with WELUX fine silica column (Intertec, Korea), the molecular weight of each ionizable lipid produced by the reaction was calculated and they were stored at a concentration of 100mg/ml using ethanol. The ionizable lipid produced by using 241 amine and C10 was named '241-C10', and other ionizable lipids produced by using other kinds of amines were named 'used amine name (241 to 246)-C10' in the same way.

**Example 1-2. Confirmation of produced ionizable lipids**

In order to confirm the ionizable lipids produced in the Example 1-1, $^1H$ NMR was performed. Specifically, the ionizable lipid (246-C10) synthesized in Example 1-1 of 5ug was prepared by diluting in $CDCl_3$ (sigma, USA) 0.5ml to 100 mmole concentration. Then, 0.5ml each was put into a tube for 400MHz NMR and the top was sealed, and then sealed with parafilm to obtain NMR spectra using Agilent 400MHZ FT-NMR (Agilent, USA), and the result was shown in FIG. 2. As shown in FIG. 2, it could be seen that the signal representing each functional group of 246-C10 was saturated.

In addition, in order to confirm the ionizable lipids (241-C10 to 246-C10) prepared in Example 1-1, MS analysis was performed. Specifically, the ionizable lipids were diluted in ethanol at a concentration of 0.5ppm or less and MS analysis was performed. The equipment used for the analysis was 6230 LC/MS of Agilent Technologies(Palo Alto,USA) and the Zorbax SB-C18 (100 mmX2.1 mm i.d., 3.5 μm) of Agilent Technologies was used for the separation tube, and two solvents of distilled water (A) containing 0.1% formic acid and acetonitrile (B) were gradient eluted. The solvent

gradient of the mobile phase was maintained for 4 minutes until the ratio of the organic solvent acetonitrile (B) was initially increased from 30% to 80% for 2 minutes and then the ratio of the organic solvent was lowered to 30% again and stabilized. The flow rate of the mobile phase was 300μℓ/min, and then, the injection volume of the analyzer was 2μℓ. The result of performing the MS analysis was shown in Table 2 below. As shown in Table 2, it could be confirmed that the measured m/z ratio and calculated m/z ratio of the ionizable lipids were almost identical.

[Table 2]

| | Chemical formula | Calculated m/z ratio | Observed m/z ratio |
|---|---|---|---|
| 241-C10 | $C_{32}H_{66}N_2O_2$ | 510.87864 | 511.5201 |
| 242-C10 | $C_{31}H_{64}N_2O_2$ | 496.85206 | 497.5043 |
| 243-C10 | $C_{31}H_{65}N_3O_2$ | 511.8667 | 513.5186 |
| 244-C10 | $C_{42}H_{87}N_3O_3$ | 682.15848 | 682.6821 |
| 245-C10 | $C_{43}H_{89}N_3O_3$ | 696.18506 | 696.7045 |
| 246-C10 | $C_{58}H_{120}N_4O_4$ | 937.5978 | 937.9383 |

From the result, it could be confirmed that the ionizable lipids were well made in

Example 1-1.

**Example 2. Preparation of lipid nanoparticles**

**Example 2-1. Preparation of lipid nanoparticles**

The ionizable lipids (241-C10 to 246-C10) prepared in the Example 1-1, cholesterol (Cholesterol powder, BioReagent, suitable for cell culture, >_99%, sigma, Korea), phospholipid (DSPC) (Avanti, US), and a lipid-PEG conjugate (ceramide-PEG conjugate; C16 PEG2000 Ceramide, Avanti, US) were dissolved in ethanol at a molar ratio of 42.5:13:43:1.5.

The ethanol in which the ionizable lipids, cholesterol, phospholipid and lipid-PEG were dissolved and acetate buffer were mixed with a microfluid mixing device (Benchtop Nanoassemblr; PNI, Canada) at a flow rate of 12ml/min in a volume ratio of 1:3, thereby preparing lipid nanoparticles (LNPs).

**Example 2-2. Preparation of nucleic acid-encapsulated lipid nanoparticles**

The ionizable lipids (241-C10 to 246-C10) prepared in the Example 1-1, cholesterol (Cholesterol powder, BioReagent, suitable for cell culture, >_99%, sigma, Korea), phospholipid (DSPC or DOPE) (18:0 PC (DSPC), 18:1 (△9-Cis) PE (DOPE), Avanti, US), and a lipid-PEG conjugate (ceramide-PEG conjugate; C16 PEG2000 Ceramid, Avanti, US) were dissolved in ethanol. An RNA therapeutic agent, mRNA (luciferase mRNA; SEQ ID NO: 1) 30μg was diluted in sodium citrate 0.75ml, or siRNA (siLUC, siGFP, Anti-HPV16 E6/E7 siRNA, Anti-PMVK siRNA etc.) 30μg was diluted in sodium acetate(50mM) 0.75ml to prepare an aqueous phase. mRNA and siRNA were synthesized and used by requesting Bioneer (Korea).

The aqueous phase (sodium acetate or sodium citrate) in which the organic phase (ethanol) in which the ionizable lipids, cholesterol, phospholipid and lipid-PEG conjugate (hereinafter, lipid-PEG) were dissolved and an RNA therapeutic agent (nucleic acid) were dissolved were mixed through a microfluid mixing device (Benchtop Nanoassemblr; PNI, Canada) at a flow rate of 12 mℓ/min, to prepare lipid nanoparticles (LNPs) in which the nucleic acid was encapsulated. (i) In order to prepare a lipid nanoparticle in which mRNA is encapsulated, the ionizable lipid : phospholipid (DOPE): cholesterol : lipid-PEG (C16-PEG2000 ceramide) were dissolved in ethanol at a molar ratio of 26.5 : 20 : 52.5 to 51 : 1.0 to 2.5 (adjusting the content of cholesterol and lipid-PEG so that the total sum of the molar ratio is 100), and the organic phase and the aqueous phase were mixed so that the mRNA (luciferase mRNA; SEQ ID NO: 1) : ionizable lipid was at the weight ratio of 1: 10, and thereby a lipid nanoparticle was prepared. (ii) In order to prepare a lipid nanoparticle in which siRNA is encapsulated, the ionizable lipid : phospholipid (DSPC): cholesterol : lipid-PEG (C16-PEG2000 ceramide) were dissolved in ethanol at a molar ratio of 42.5 : 13 : 44 to 39.5 : 0.5 to 5.0 (adjusting the content of cholesterol and lipid-PEG so that the total sum of the molar ratio is 100), and the organic phase and the aqueous phase were mixed so that the siRNA (siFVII; SEQ ID NOs: 2 and 3 were mixed at the same molar ratio, or siFVIII; SEQ ID NOs: 4 to 11 were mixed at the same molar ratio, or siLuc: SEQ ID NOs: 12 and 13 were mixed at the same ratio): ionizable lipid was

at the weight ratio of 1:7.5 and thereby a lipid nanoparticle (LNP) was prepared. The used siRNA sequences were described in Table 3 below. In Table 3, bases indicated by lowercase letters were modified with 2'-O-Methyl.

[Table 3]

| | | Base sequence (5'->3') | SEQ ID NO: |
|---|---|---|---|
| siLuc | sense | AAcGcuGGGcGuuAAucAAdTdT | SEQ ID NO: 2 |
| | antisense | UUGAUuAACGCCcAGCGUUdTdT | SEQ ID NO: 3 |
| Cy5.5 labeled siGFP | sense | ACAUGAAGCAGCACGACUUdTdT | SEQ ID NO: 4 |
| | antisense | AAGUCGUGCUGCUUCAUGUdTdT | SEQ ID NO: 5 |
| Anti-HPV16 E6/E7 siRNA | sense | GACCGGUCGAUGUAUGUCUUG | SEQ ID NO: 6 |
| | antisense | AGACAuACAuCGACCGGuCCA | SEQ ID NO: 7 |
| Anti-PMVK siRNA | sense | UGGACGAUGCUGAGUCAGAdTdT | SEQ ID NO: 14 |
| | antisense | UCUGACUCAGCAUCGUCCAdTdT | SEQ ID NO: 15 |

The prepared LNPs were dialyzed against PBS for 16 hours using a 10,000 MWCO dialysis cassette to remove ethanol and adjust the body pH and the pH of the nanoparticles.

The lipid nanoparticles comprising the ionizable lipid '241-C10' were named '241-C10 LNP', and the lipid nanoparticles prepared by using the ionizable lipid comprising amine (including lipid nanoparticles in which a nucleic acid was encapsulated) were named 'comprised amine name (241 to 246)-C10 LNP' .

**Example 2-3. Observation of nucleic acid-encapsulated lipid nanoparticles**

The Lipid nanoparticles in which siLuc (SEQ ID NO: 5) were encapsulated was prepared by using a ceramide-PEG conjugate (C16-PEG2000 ceramide) as Example 2-2. The prepared lipid nanoparticles (comprising 1.5 mol% of ceramide-PEG conjugate) were loaded on 200 mesh carbon lacey film Cu-grid in an amount of 60ug based on siRNA concentration and were immersed in ethane liquefied with vitrobot (about -170 degrees or less) and were plunge frozen to be prepared, and then were observed with Cryo-TEM (Tecnai F20, FEI), and the result was shown in FIG. 1b. As shown in FIG. 1b, spherical particles with a solid shape were observed.

**Example 3. pKa of lipid nanoparticles**

In the present example, pKa of each lipid nanoparticle (LNP) formulated in the Example 2-1 was calculated through In vitro TNS assay. Anionic TNS becomes lipophilic by interacting with a positively charged ionizable lipid, and as the pH value becomes close to the pKa value of each LNP, the lipophilic property of TNS becomes lower and more water molecules quench the TNS fluorescence, and therefore, lipid nanoparticles having a pKa of 6.0 to 7.0 have excellent in vivo drug delivery efficiency, and lipid nanoparticles showing a "s-type curve" in the graph representing fluorescence according to pH mean that they are easy to interact with the endosome membrane and can easily escape the endosome during acidification.

Specifically, the pH of the solution comprising 20mM sodium phosphate, 25mM citrate, 20mM ammonium acetate, and 150mM NaCl with 0.1N NaOH and/or 0.1N HCl at an interval of 0.5 from pH 4.1 to pH 9.6 to prepare solutions of various pH units. 100$\mu\ell$ of each solution having each pH (pH with an interval of 0.5 from pH 4.1 to pH 9.6) was added to a black 96well plate and each was added to a solution having the pH in the range so as to be the final concentration of 6uM using a TNS stock solution of 300uM. 241-C10 LNP to 246-C10 LNP were added to the mixed solution so that the final concentration is 20uM. The fluorescence intensity was measured by excitation at 325 nm and emission at 435 nm through a Tecan equipment, and the fluorescence intensity for each lipid nanoparticle was shown in FIG. 3a and FIG. 3b, and the pKa for each lipid nanoparticle was calculated as a pH value reaching half of the maximum fluorescence and shown in Table 4 below. As shown in FIG. 3b, it could be seen that 244-C10 LNP to 246-C10 LNP exhibit a fluorescence titration s-shaped curve through nonlinear regression.

[Table 4]

| Lipid nanoparticles | pKa |
|---|---|
| 241-C10 LNP | 7.7 |

(continued)

| Lipid nanoparticles | pKa |
|---|---|
| 242-C10 LNP | 8.7 |
| 243-C10 LNP | 8.2 |
| 244-C10 LNP | 6.8 |
| 245-C10 LNP | 6.9 |
| 246-C10 LNP | 7 |

As confirmed in the Table 4, it was confirmed that the lipid nanoparticles according to one example showed pKa 6.0 to 7.0 range in which in vivo safety and drug release are excellent.

The LNPs in which a nucleic acid was encapsulated, prepared by the method as Example 2-2, also showed the same pattern according to the type of ionizable lipids contained (type of amine contained in the ionizable lipids).

## Example 4. Confirmation of characteristics of lipid nanoparticles

### Example 4-1. Particle size measurement

In the present example, the size of the lipid nanoparticles (LNP; comprising 1.5 mol% of lipid-PEG) in which mRNA was encapsulated measured in Example 2-2 was to be measured. It was diluted using PBS so that the concentration of RNA (luciferase mRNA; SEQ ID NO: 1) comprised in each lipid nanoparticle prepared in Example 2-2 was 1μg/ml, and the diameter and polydispersity index (PDI) of the LNPs were measured using dynamic light scattering (DLS) in Malvern Zetasizer Nano (Malvern Instruments, UK), and the result was described in Table 5 below.

[Table 5]

| Lipid nanoparticles | Diameter (nm) | PDI |
|---|---|---|
| 241-C10 LNP | 128 | 0.259 |
| 242-C10 LNP | 77 | 0.210 |
| 243-C10 LNP | 56 | 0.225 |
| 244-C10 LNP | 66 | 0.149 |
| 245-C10 LNP | 70 | 0.210 |
| 246-C10 LNP | 68 | 0.143 |

As confirmed in the Table 5, the lipid nanoparticles according to one example showed the particle size that is easy to be introduced into hepatocytes and has excellent drug release, and it could be found that the PDI values were small and the particles were uniform in order of 241-C10 LNP > 243-C10 LNP > 242-C10 LNP = 245-C10 LNP > 244-C10 LNP > 246-C10 LNP.

### Example 4-2. Measurement of encapsulation efficiency

The encapsulation efficiency (drug encapsulation efficiency, %) of each LNP (comprising 1.5 mol% of lipid-PEG) in which siRNA (siFVII siRNA) was encapsulated as a nucleic acid drug was measured through Ribogreen analysis (Quant-iT™ RiboGreen® RNA, Invitrogen). The LNPs in which a nucleic acid drug was encapsulated prepared in the Example 2-2 were diluted with 1xTE buffer solution 50μℓ in a 96 well plate so that the final concentration of siRNA was 4 ~ 7 μg/ml. To the group untreated with Triton-X (Triton-x LNP(-)), 1xTE buffer 50μℓ was added, and to the group treated with Triton-X (Triton-x LNP(+)), 2% Triton-X buffer 50μℓ was added. By incubating at 37°C for 10 minutes, the nucleic acid encapsulated by degrading LNPs with Triton-X was released. Then, Ribogreen reagent 100μℓ was added to each well. The fluorescence intensity (FL) of Triton LNP(-) and Triton LNP(+) was measured by the wavelength bandwidth (excitation : 485nm, emission : 528nm) in Infinite® 200 PRO NanoQuant (Tecan), and the drug encapsulation efficiency (encapsulation efficiency, %) was calculated as the following Equation 3. The drug encapsulation efficiency (%) for each LNP was shown in Table 6 below as the average value of the results measured repeatedly twice.

(Equation 3)

Drug encapsulation efficiency (%)=(Fluorescence intensity of Triton LNP(+) – Fluorescence intensity of Triton LNP(-))/(Fluorescence intensity of Triton LNP(+)) X 100

[Table 6]

| Lipid nanoparticles | Encapsulation efficiency (%) |
|---|---|
| 241-C10 LNP | 84 |
| 242-C10 LNP | 83 |
| 243-C10 LNP | 91 |
| 244-C10 LNP | 87 |
| 245-C10 LNP | 91 |
| 246-C10 LNP | 94 |

As confirmed in the Table 6, it was confirmed that the lipid nanoparticles according to one example could encapsulate a drug with high efficiency.

**Example 5. Confirmation of intracellular nucleic acid delivery using lipid nanoparticles**

**Example 5-1. Nucleic acid delivery effect according to types of ionizable lipids comprised in LNP**

One day prior to transfection of LNP according to one example into cells, HeLa cells (Korea Cell Line Bank) were aliquoted at $0.01\times10^6$ cells/well in a white plate (96well) and were cultured under the condition of 37°C, 0.5~3% $CO_2$ in DMEM media (SH30022, Hyclone, USA). After stirring LNPs (241-C10 LNP to 246-C10 LNP comprising 1.5mol% of lipid-PEG) in which mRNA (luc mRNA; SEQ ID NO: 1) encoding a luciferase gene with ApoE3 0.1μg/ml by pipetting and then incubating at a room temperature for 10 minutes, they were treated (100ng/well based on the mRNA comprised in the lipid nanoparticles) in HeLa cells. ApoE3 binds to the LNP surface and plays a role in allowing LNP to enter the cell through endocytosis through an LDL receptor expressed on the cell surface.

In 24 hours, after treating 100μℓ/well of Bright-Glo™ Luciferase Assay solution (promega, USA) each and leaving them at a room temperature for 10 minutes, the luminescence intensity was measured for the dissolved cells using Infinite M200 luminescence measuring device (Tecan, USA), and the result was shown in FIG. 4a. As shown in FIG. 4a, 244-C10 LNP, 245-C10 LNP, and 246-C10 LNP having a pKa range of 6.0 to 7.0 showed strong luminescence intensity, and among them, 246-C10 LNP had the highest luminescence intensity, and therefore, it could be seen that 246-C10 LNP had the highest intracellular drug delivery efficiency.

**Example 5-2. Confirmation of nucleic acid delivery in cancer cells**

Lipid nanoparticles were prepared by changing the content of the lipid-PEG by comprising C16 PEG-ceramide or PEG-DSPE in the lipid nanoparticles similarly to the method of Example 2-2.

The weight ratio of the ionizable lipid: siRNA comprised in the lipid nanoparticle was 7.5 : 1, and the molar ratio of the ionizable lipid (246-C10): phospholipid (DSPC): cholesterol: lipid-PEG (C16-PEG2000 ceramide or PEG-DSPE) comprised in the LNP was 42.5 : 13 : 39.5 to 43 : 1.5 to 5.0 (the contents of cholesterol and lipid-PEG was adjusted so that the sum of the molar ratios was 100).

One day prior to transfection of LNP according to one example into cells, HeLa cells (Korea Cell Line Bank) were aliquoted at $0.01\times10^6$ cells/well in a white plate (96well) and were cultured under the condition of 37°C, 0.5~3% $CO_2$ in DMEM media (SH30022, Hyclone, USA). In 24 hours after the lipid nanoparticles in which siLuc (SEQ ID NOs: 2 and 3) were encapsulated were treated into HeLa-Luc cell line at a siRNA concentration of 10 nM, Bright-Glo™ Luciferase Assay solution (promega, USA) was treated by 100μℓ/well each, and it was left at a room temperature for 10 minutes, and then, the luminescence intensity of the dissolved cells was measured using Infinite M200 luminescence measuring device (Tecan, USA), and the result was shown in FIG. 4b. The sequence of the used siLuc (siRNA targeting

a luciferase gene) was described in Table 3 above.

As shown in FIG. 4b, the lipid nanoparticle according to one example had an excellent nucleic acid delivery effect into Hela-Luc cells, which are cancer cells.

**Example 5-3. Pharmacokinetic Analysis**

Lipid nanoparticles in which Anti-HPV16 E6/E7 siRNA (SEQ ID NOs: 6 and 7; Table 3) were encapsulated as the content of the lipid-PEG comprised in the lipid nanoparticle was 1.5 to 5.0 mol% as the method of Example 2-2. The weight ratio of the ionizable lipid: siRNA comprised in the lipid nanoparticle was 7.5 : 1, and the molar ratio of the ionizable lipid (246-C10): phospholipid (DSPC): cholesterol: lipid-PEG (C16-PEG2000 ceramide) comprised in the LNP was 42.5 : 13 : 39.5 to 43 : 1.5 to 5.0. The diameter and PDI of the lipid nanoparticles prepared above were measured as same as the method of Example 4-1 and shown in Table 7 below.

[Table 7]

| Lipid-PEG ( mol%) | Average diameter (nm) | PDI |
|---|---|---|
| 5.0 | 67.4 | 0.312 |
| 1.5 | 122.9 | 0.214 |

After injecting 2 mg/kg drug of the lipid nanoparticle in which anti-HPV16 E6/E7 siRNA was encapsulated into the femoral vein of Spragu-Dawley rats (7-8 weeks old, male), blood was collected for 0.5 minutes ~ 24 hours, and immediately, after centrifugation at 13,000 rpm at 4°C for 5 minutes, plasma was secured and stored in a -70°C deep freezer. Anti-HPV16 E6/E7 siRNA was confirmed in the plasma using a stem-loop RT-qPCR detection method from the secured plasma. When stem-loop RT primers and probes specific to siRNA were selected and a real time PCR technique was applied using a siRNA standard sample, as a result of linear analysis between Cp values (y) for the siRNA standard sample, recurrence between the siRNA standard sample and Cp values was analyzed with the slope average and determination coefficient $R^2$ to performed the stem-loop RT-qPCR detection method. The result of analyzing pharmacokinetic parameters using WinNonlin program was shown in Table 8.

[Table 8]

| | Naked | 246C10 Lipid (Lipid-PEG 5%) | 246C10 Lipid (Lipid-PEG 1.5%) |
|---|---|---|---|
| T max (min) | 1.000 | 1.000 | 30.000 |
| C max (ng/mL) | 146025.200 | 473280.700 | 289864.600 |
| T 1/2 (min) | 3.384 | 87.321 | 588.182 |
| CL (mL/min/kg) | 2.020 | 0.034 | 0.006 |

As shown in Table 8, in the LNP in which siHPV16_E6/E7 was encapsulated, compared to naked siRNA, the maximum concentration in blood (Cmax) after drug administration was higher, and the drug elimination half-life (T1/2) was maintained longer, and the clearance rate (CL) of the drug was lower, so it was maintained in the body for a long time, and the degree of eliminating the drug was low, and therefore, the in vivo stability was increased.

**Example 5-4. Confirmation of migration of siRNA-LNPs into cancer tissue**

Lipid nanoparticles (246-C10 LNP) in which Cy5.5 labeled siGFP (SEQ ID Nos: 4 and 5; Table 3) was encapsulated were prepared as the content of the lipid-PEG comprised in the lipid nanoparticle was 5.0 mol%. The weight ratio of the ionizable lipid: siRNA comprised in the lipid nanoparticle was 7.5 : 1. and the molar ratio of the ionizable lipid (246-C10): phospholipid (DSPC): cholesterol: lipid-PEG (C16-PEG2000 ceramide) comprised in the LNP was 42.5 : 13 : 39.5 : 5.0. The diameter and PDI of the lipid nanoparticles prepared above were measured as same as the method of Example 4-1 and shown in Table 9 below.

[Table 9]

| Lipid-PEG (%) | Average diameter (nm) | PDI |
|---|---|---|
| 5.0 | 30 | 0.142 |

$5\times10^6$ HeLa cells were subcutaneously inoculated into C57BL/6 female 7-week-old 20g mice. The tumor volume and body weight of the mice were monitored twice a week. The tumor size was measured using digital calipers. The volume of the tumor was calculated using $V = 0.5 \times W^2 \times L$ (V = tumor volume, W = tumor width, L = tumor length). When the tumor size reached $100mm^3$, the lipid nanoparticles were injected into the mice, and in 48 hours after injection, biodistribution was confirmed by IVIS. The fluorescence in cancer tissue, liver and kidney was measured, and the result was shown in FIG. 5a. As shown in FIG. 5a, it could be confirmed that migration into cancer tissue was excellent when the lipid nanoparticle according to one example was administered.

### Example 5-5. Confirmation of in vivo siRNA delivery and gene regulation in xenograft tumor mouse model

As confirmed in Example 5-2 above, the in vivo drug delivery efficiency and biodistribution of 246-C10 LNP showing an excellent gene expression effect (gene delivery effect) in vitro were to be confirmed in the present example.

By the method of Example 5-4, siLuc (SEQ ID Nos: 2 and 3; Table 3) was prepared with 246-C10 LNP (comprising lipid-PEG of 1.5 mol%), and each nanoparticle was dialyzed in PBS for 16 hours to remove ethanol. $5\times10^6$ HeLa-Luc cancer cells were subcutaneously inoculated into C57BL/6 Female 7-week-old (Orient Bio) 20g mice. The tumor volume and body weight of the mice were monitored twice a week. The tumor volume and body weight of the mice were monitored twice a week. The tumor size was measured using digital calipers. The volume of the tumor was calculated using $V = 0.5 \times W^2 \times L$ (V = tumor volume, W = tumor width, L = tumor length). When the tumor size reached $100mm^3$, the siLuc lipid nanoparticles and Negative control siRNA were injected into the mice, and in 48 hours after injection, bioluminescence intensity was shown using IVIS (PerkinElmer, USA) equipment. As shown in FIG. 5b, it could be confirmed that the luminescence intensity was reduced in the group in which the complementary siLuc lipid nanoparticle was administered in vivo of the HeLa-Luc xenograft mice, compared to the group in which the negative control siRNA lipid nanoparticle was administered. Through this, it could be confirmed that the delivery and conservative force of the lipid nanoparticle according to one example were excellent.

By the method of Example 5-4, siLuc (SEQ ID NOs: 2 and 3; Table 3) was prepared with 246-C10 LNP (comprising lipid-PEG of 1.5 mol%), and each nanoparticle was dialyzed in PBS for 16 hours to remove ethanol. The bioluminescence intensity according to the injection method was measured by manufacturing a Xenograft mouse model by the same method as FIG. 5b.

In 3 hours after injecting the lipid nanoparticle in which siLuc was encapsulated by a method of IV (intravenous) injection or IT (intratumoral) injection, bioluminescence was confirmed by IVIS (PerkinElmer, USA) equipment, and the result was shown in FIG. 5c. As shown in FIG. 5c, the bioluminescence according to the injection method in which the lipid nanoparticle comprising siLuc was administered was plotted, and it could be confirmed that both intravenous injection and intratumoral injection reduced the luminescence intensity.

### Example 6. Confirmation of cancer treatment effect using Anti-HPV16 E6/E7 siRNA

### Example 6-1. Preparation of siRNA targeting HPV16 E6/E7 gene-encapsulated lipid nanoparticles

The 246-C10 lipid nanoparticles (comprising lipid-PEG of 1.5 mol%) in which siRNA targeting E6/E7 genes of HPV16 (SEQ ID NOs: 6 and 7; Table 3) was encapsulated were prepared similarly to the method of Example 2-2.

### Example 6-2. Cancer cell killing effect measurement

In order to measure the killing effect of cancer cells, into a head and neck squamous carcinoma carcinoma (HNSCC) cell line, UM-SCC-47 (Meark Cat No. SCC071) cells, and a cervical cancer cell line, Ca Ski (ATCC Cat No. CRL-1550) cell line, the LNP in which the Anti-HPV16 E6/E7 siRNA prepared in Example 6-1 was encapsulated was transfected.

The head and neck squamous carcinoma carcinoma cell line, UM-SCC-47 was cultured in a DMEM medium comprising 10% fetal bovine serum and an antibiotic (Hyclone company Cat: SH30243.01) under conditions of 37°C, 5% $CO_2$ and 100% humidity. The cervical cancer cell line, Ca Ski was cultured in an RPMI (Hyclone company Cat: SH30027.01) medium comprising 10% fetal bovine serum and an antibiotic under conditions of 37°C, 5% $CO_2$ and 100% humidity.

$1\times10^5$ cells were put in a 6 well plate and cultured before 24 hours of transfection, and then, the Anti HPV16 E6/E7 siRNA-encapsulated 246-C10 LNP (comprising lipid-PEG of 1.5 mol%) was treated at a concentration of 10 ~ 30nM into UM-SCC-47, and was treated at a concentration of 10~40nM into Ca Ski cells, in the single administration group and the combination administration group, and in case of cisplatin (CDDP), in the administration group and the combination administration group, it was treated at a concentration of 1~5uM in UM-SCC-47, and at a concentration of 5~10uM in Ca Ski, and combined with Apoe4 1.5ug/ml at a room temperature for 15 minutes, and then treated in a 6

well plate. In 48 hours after transfection, the cell viability was confirmed by confirming the staining degree of trypan blue in the cells, with a phase-contrast microscope using a hemocytometer, and the result was shown in FIG. 6a.

As shown in FIG. 6a, the cancer cell killing effect by the siRNA-encapsulated lipid nanoparticle according to one example was excellent, and when used in combination with the anticancer agent (CDDP), the killing effect was further increased.

**Example 6-3. mRNA expression measurement**

In the present example, in order to measure the drug (siRNA) delivery efficiency by the lipid nanoparticle according to one example, mRNA expression of HPV 16 E6/E7 was measured, and in order to confirm the effect of cancer prevention or treatment by the lipid nanoparticle according to one example, p21 mRNA expression was measured.

In order to measure the mRNA expression level, into a head and neck squamous carcinoma carcinoma (HNSCC) cell line, UM-SCC-47 (Meark Cat No. SCC071) cells, and a cervical cancer cell line, Ca Ski (ATCC Cat No. CRL-1550) cell line, the Anti-HPV16 E6/E7 siRNA-encapsulated LNP prepared in Example 6-1 was transfected. The head and neck squamous carcinoma carcinoma cell line, UM-SCC-47 and cervical cancer cell line, Ca Ski were cultured as Example 6-2.

$1 \times 10^5$ cells were put in a 6 well plate and cultured before 24 hours of transfection, and then, the Anti HPV16 E6/E7 siRNA-encapsulated 246-C10 LNP (comprising lipid-PEG of 1.5 mol%) was treated at a concentration of 10 ~ 30nM into UM-SCC-47, and was treated at a concentration of 10~40nM into Ca Ski cells, in the single administration group and the combination administration group, and in case of cisplatin (CDDP), in the administration group and the combination administration group, it was treated at a concentration of 1~5uM in UM-SCC-47, and at a concentration of 5~10uM in Ca Ski, and combined with Apoe4 1.5ug/ml at a room temperature for 15 minutes, and then treated in a 6 well plate.

In 48 hours after transfection, cells were lysed, and RNA was extracted according the manufacture's instructions using RiboEX ™(GeneAll, Cat no 301-001), and Quantitative Real time - Polymerase chain Reaction (qRT-PCR) capable of confirming the expression level of mRNA of HPV16 E6/E7 (Forward primer: SEQ ID NO: 8 / Reverse primer: SEQ ID NO: 9/Probe : UPL#63 (Roche)), P21 (Forward primer: SEQ ID NO: 101 Reverse primer: SEQ ID NO: 11/ Probe : UPL#82 (Roche)), HPRT1 (Forward primer: SEQ ID NO: 12 / Reverse primer: SEQ ID NO: 131 Probe : UPL#73(Roche)) was performed, and the result was shown in FIG. 6b. HPRT1 was used as a house keeping gene, and the used primer sequences were described in Table 10 below.

[Table 10]

| Primer | | Base sequence (5'->3') | SEQ ID NO: |
|---|---|---|---|
| HPV16_E6/E7 | Forward | CCACTGATCTCTACTGTTATGAGCAA | SEQ ID NO: 8 |
| | Reverse | CCAGCTGGACCATCTATTTCA | SEQ ID NO: 9 |
| P21 | Forward | CGAAGTCAGTTCCTTGTGGAG | SEQ ID NO: 10 |
| | Reverse | CATGGGTTCTGACGGACAT | SEQ ID NO: 11 |
| HPRT1 | Forward | TGACCTTGATTTATTTTGCATACC | SEQ ID NO: 12 |
| | Reverse | CGAGCAAGACGTTCAGTCCT | SEQ ID NO: 13 |

As shown in FIG. 6b, it could be confirmed that when the Anti-HPV16 E6/E7 siRNA-encapsulated lipid nanoparticle was treated, the mRNA expression of HPV16 E6/E7 was significantly reduced, and the expression of p21 mRNA, a subgene of a tumor suppressor gene p53, was significantly increased, and when used in combination with a chemical anticancer agent (Cisplatin, CDDP), the mRNA expression decrease of HPV16 E6/E7 and p21 mRNA expression increase were further increased.

**Example 6-4. Protein expression measurement**

In the present example, in order to confirm the effect of cancer prevention or treatment by the lipid nanoparticle according to one example, p21 and p53 protein expression was measured.

In order to measure the protein expression level, into a head and neck squamous carcinoma carcinoma (HNSCC) cell line, UM-SCC-47 (Meark Cat No. SCC071) cells, and a cervical cancer cell line, Ca Ski (ATCC Cat No. CRL-1550) cell line, the Anti-HPV16 E6/E7 siRNA-encapsulated LNP prepared in Example 6-1 was transfected. The head and neck squamous carcinoma carcinoma cell line, UM-SCC-47 and cervical cancer cell line, Ca Ski were cultured as Example 6-2.

$1 \times 10^5$ cells were put in a 6 well plate and cultured before 24 hours of transfection, and then, the Anti HPV16

E6/E7 siRNA-encapsulated 246-C10 LNP (comprising lipid-PEG of 1.5 mol%) was treated at a concentration of 10 ~ 30nM into UM-SCC-47, and was treated at a concentration of 10~40nM into Ca Ski cells, in the single administration group and the combination administration group, and in case of cisplatin (CDDP), in the administration group and the combination administration group, it was treated at a concentration of 1~5uM in UM-SCC-47, and at a concentration of 5~10uM in Ca Ski, and combined with Apoe4 1.5ug/ml at a room temperature for 15 minutes, and then treated in a 6 well plate.

In 48 hours after transfection, using a western blot analysis method, the expression levels of p21, p53, and a house keeping protein, b-actin were measured. They were lysed by adding RIPA cell lysis buffer (150mM NaCl, 10 mM Tris-HCl (Ph7.4), 5 mM EDTA, 0.1% SDS, 0.5% deoxycholate and 1% NP-40), and western blot was conducted with the same protein amount through BCA assay capable of quantifying the amount of protein. The primary antibody Anti-P21 (Santacruz Cat no. SC-817) was used as diluted by 1:2000, and Anti-p53 (Santacruz Cat no. SC-47698) was used as diluted by 1:2000, and Anti-B-actin (Santacruz Cat no. SC-47778) was used as diluted by 1:3000. It was performed by diluting an IgG-Mouse-HRP-bound antibody as a secondary antibody by 1:5000, and the result was shown in FIG. 6c.

As shown in FIG. 6c, when the Anti-HPV16 E6/E7 siRNA-encapsulated lipid nanoparticle was treated alone or in combination with an anticancer agent (CDDP), p21 and p53 protein expression was significantly increased.

**Example 7. Confirmation of cancer treatment effect using PMVK siRNA**

**Example 7-1. Preparation of lipid nanoparticles in which siRNA targeting PMVK is encapsulated**

In a similar way to the method of Example 2-2, the 246-C10 lipid nanoparticles (comprising lipid-PEG of 1.5 mol%) in which siRNA targeting PMVK (SEQ ID NOs: 14 and 15; Table 3) was encapsulated were prepared.

**Example 7-2. Cancer cell killing effect measurement**

In order to measure the killing effect of cancer cells, into a pancreatic cancer cell line, Miapaca-2 (ATCC Cat.no CL-1420), a lung cancer cell line, A549 (ATCC Cat.no CCL-185), a breast cancer cell line, MCF-7 (ATCC Cat.no HTB-22), and a cervical cancer cell line, Hela (ATCC Cat.no CCL-2) cell line, the Anti-PMVK siRNA-encapsulated LNP prepared in Example 7-1 was transfected.

The pancreatic cancer cell line, Miapaca-2, and the cervical cancer cell line, Hela were cultured in a DMEM medium comprising 10% fetal bovine serum and an antibiotic (Hyclone company Cat: SH30243.01) under conditions of 37°C, 5% $CO_2$ and 100% humidity.

The lung cancer cell line, A549 and the breast cancer cell line, MCF-7 were cultured in an RPMI medium comprising 10% fetal bovine serum and an antibiotic (Hyclone company Cat: SH30027.01) under conditions of 37°C, 5% $CO_2$ and 100% humidity.

$1\times10^5$ cells were put in a 6 well plate and cultured before 24 hours of transfection, and then, the Anti PMVK siRNA-encapsulated 246-C10 LNP (comprising lipid-PEG of 1.5 mol%) was treated at a concentration of 20~40nM in the single administration group and the combination administration group, and in case of cisplatin ((CDDP) Sigma, Cat no. P4394), in the administration group and the combination administration group, it was treated at a concentration of 1~SuM, and combined with Apoe4 1.5ug/ml at a room temperature for 15 minutes, and then treated in a 6 well plate. In 48 hours after transfection, the cell viability was confirmed by confirming the staining degree of trypan blue in the cells, with a phase-contrast microscope using a hemocytometer, and the result was shown in FIG. 7a.

As shown in FIG. 7a, the cancer cell killing effect by siRNA by the drug-encapsulated lipid nanoparticle according to one example was increased.

**Example 7-3. mRNA expression measurement**

In the present example, in order to measure the drug (siRNA) delivery efficiency by the lipid nanoparticle according to one example, the PMVK mRNA expression was measured in the lung cancer, pancreactic cancer, breast cancer, and cervical cancer cells.

In order to measure the expression level of mRNA, into a pancreatic cancer cell line, Miapaca-2 (ATCC Cat.no CL-1420), a lung cancer cell line, A549 (ATCC Cat.no CCL-185), a breast cancer cell line, MCF-7 (ATCC Cat.no HTB-22), and a cervical cancer cell line, Hela (ATCC Cat.no CCL-2) cell line, the Anti-PMVK siRNA-encapsulated LNP prepared in Example 7-1 was transfected. The pancreatic cancer cell line, Miapaca-2, cervical cancer cell line, Hela, lung cancer cell line, A549 and breast cancer cell line, MCF-7 were cultured as Example 7-2.

$0.5\sim1\times10^5$ cells were put in a 6 well plate and cultured before 24 hours of transfection, and then, the Anti PMVK siRNA-encapsulated 246-C10 LNP (comprising lipid-PEG of 1.5 mol%) was treated at a concentration of 20~40nM in the single administration group and the combination administration group, and in case of cisplatin (((CDDP) Sigma,

Cat no. P4394), in the administration group and the combination administration group, it was treated at a concentration of 1~SuM, and combined with Apoe4 1.5ug/ml at a room temperature for 15 minutes, and then treated in a 6 well plate. In 48 hours after transfection, cells were lysed, and RNA was extracted according the manufacture's instructions using RiboEX ™(GeneAll, Cat no 301-001), and Quantitative Real time - Polymerase chain Reaction (qRT-PCR) capable of confirming the expression level of mRNA of PMVK (Forward : : SEQ ID NO: 16 / Reverse : SEQ ID NO: 17 / Probe : UPL#49 (Roche)), B-ACTIN (Forward : SEQ ID NO: 18 / Reverse : SEQ ID NO: 19 / Probe : UPL#64(Roche)) was performed, and the result was shown in FIG. 7b. B-ACTIN was used as a house keeping gene, and the used primer sequences were described in Table 11 below.

[Table 11]

| Primer | | Base sequence (5'->3') | SEQ ID NO: |
|---|---|---|---|
| PMVK | Forward | ATATCCCCAGTGCCAGTCC | SEQ ID NO: 16 |
| | Reverse | AACAAGGGGCTGAGAACATC | SEQ ID NO: 17 |
| B-ACTIN | Forward | CCAACCGCGAGAAGATGA | SEQ ID NO: 18 |
| | Reverse | CCAGAGGCGTACAGGGATAG | SEQ ID NO: 19 |

As shown in FIG. 7b, when the PMVK siRNA-encapsulated lipid nanoparticle was treated into cancer cells, the mRNA expression of PMVK was significantly reduced.

**Example 7-4. Protein expression measurement**

In the present example, in order to measure the drug (siRNA) delivery efficiency by the lipid nanoparticle according to one example, the PMVK protein expression was measured in the lung cancer, pancreactic cancer, breast cancer, and cervical cancer cells.

In order to measure the expression level of protein, into a pancreatic cancer cell line, Miapaca-2 (ATCC Cat.no CL-1420), a lung cancer cell line, A549 (ATCC Cat.no CCL-185), a breast cancer cell line, MCF-7 (ATCC Cat.no HTB-22), and a cervical cancer cell line, Hela (ATCC Cat.no CCL-2) cell line, the Anti-PMVK siRNA-encapsulated LNP prepared in Example 7-1 was transfected. The pancreatic cancer cell line, Miapaca-2, cervical cancer cell line, Hela, lung cancer cell line, A549 and breast cancer cell line, MCF-7 were cultured as Example 7-2.

0.5~1$\times$10$^5$ cells were put in a 6 well plate and cultured before 24 hours of transfection, and then, the Anti PMVK siRNA-encapsulated 246-C10 LNP (comprising lipid-PEG of 1.5 mol%) was treated at a concentration of 20~40nM in the single administration group and the combination administration group, and in case of cisplatin (((CDDP) Sigma, Cat no. P4394), in the administration group and the combination administration group, it was treated at a concentration of 1~SuM, and combined with Apoe4 1.5ug/ml at a room temperature for 15 minutes, and then treated in a 6 well plate. In 48 hours after transfection, using a western blot analysis method, the expression level of PMVK was measured. They were lysed by adding RIPA cell lysis buffer (150mM NaCl, 10 mM Tris-HCl (Ph7.4), 5 mM EDTA, 0.1% SDS, 0.5% deoxycholate and 1% NP-40), and western blot was conducted with the same protein amount through BCA assay capable of quantifying the amount of protein. The primary antibody Anti-PMVK(Atlas antibody Cat no. HPA029900) was used as diluted by 1:2000. It was performed by diluting an IgG-Mouse-HRP-bound antibody as a secondary antibody by 1:5000, and the result was shown in FIG. 7c.

As shown in FIG. 7c, when the Anti-PMVK siRNA-encapsulated lipid nanoparticle was treated into cancer cells, the PMVK protein expression was significantly reduced.

**Example 7-5. Viability measurement by raditation sensitivity upon PMVK knock-down in cance cell line**

In the present example, in order to measure the drug (siRNA) delivery efficiency by the lipid nanoparticle according to one example, the cell viability upon PMVK knock-down known as a factor related to radiotherapy resistance in lung cancer, pancreactic cancer, and cervical cancer cells was measured.

In order to measure the expression level of protein, into a pancreatic cancer cell line, Miapaca-2 (ATCC Cat.no CL-1420), a lung cancer cell line, A549 (ATCC Cat.no CCL-185), a cervical cancer cell line, Hela (ATCC Cat.no CCL-2) cell line, and Ca Ski (ATCC Cat.no CRL-1550) cell line, the 246-C10 lipid nanoparticle (comprising lipid-PEG of 1 mol%) in which siRNA targeting the PMVK gene (SEQ ID NOs: 14 and 15; Table 3) was encapsulated similarly to the method of Example 2-2 was transfected. The pancreatic cancer cell line, Miapaca-2, cervical cancer cell line, Hela, Ca Ski, and lung cancer cell line, A549 were cultured as Example 7-2.

Before 24 hours of transfection, 0.3 $\times$ 10$^4$ of Miapaca-2, A549, HeLa and Ca Ski cells were seeded in a 96

well plate, and then cultured for 24 hours. The Anti-PMVK siRNA-encapsulated 246-C10 lipid nanoparticle prepared in Example 7-5 (comprising lipid-PEG of 1 mol%) was transfected into the cultured cells. In addition, using a 6-MV photon beam linear accelerator, radiation of 10 Gy was treated. On the second day after treatment of radiation, the cell viability was confirmed through CCK-8 assay. The result was shown in FIG. 7d.

As shown in FIG. 7d, when the Anti-PMVK siRNA-encapsulated 246-C10 lipid nanoparticle and radiation were treated into the cancer cells, the cell death was significantly increased.

**Example 7-6. Protein expression measurement by raditation sensitivity upon knock-down by PMVK nanoparticle in cance cell line**

In the present example, in order to measure the drug (siRNA) delivery efficiency by the lipid nanoparticle according to one example, the protein expression upon PMVK knock-down known as a factor related to radiotherapy resistance in lung cancer, pancreactic cancer, and cervical cancer cells was measured.

In order to measure the protein expression, into a pancreatic cancer cell line, Miapaca-2 (ATCC Cat.no CL-1420), a lung cancer cell line, A549 (ATCC Cat.no CCL-185), a cervical cancer cell line, Hela (ATCC Cat.no CCL-2) cell line, and Ca Ski (ATCC Cat.no CRL-1550) cell line, the Anti-PMVK siRNA-encapsulated lipid nanoparticle prepared in Example 7-5 was transfected. The pancreatic cancer cell line, Miapaca-2, cervical cancer cell line, Hela, Ca Ski, and lung cancer cell line, A549 were cultured as Example 7-2.

Before 24 hours of transfection, $1.0 \times 10^5$ of Miapaca-2, A549, HeLa and Ca Ski cells were seeded in a 96 well plate, and then cultured for 24 hours. The Anti-PMVK siRNA-encapsulated 246-C10 lipid nanoparticle prepared in Example 7-5 (comprising lipid-PEG of 1 mol%) was transfected into the cultured cells. In addition, using a 6-MV photon beam linear accelerator, radiation of 10 Gy was treated. On the second day after treatment of radiation, protein was extracted and then western blotting was performed using a PMVK antibody, thereby confirming expression of protein. The result was shown in FIG. 7e.

As shown in FIG. 7e, it could be confirmed that when the Anti-PMVK siRNA-encapsulated 246-C10 lipid nanoparticle and radiation were treated into the cancer cells, the protein expression by PMVK siRNA was reduced.

**Example 7-7. Confirmation of in vivo siRNA delivery and gene regulation by nanoparticle comprising siRNA in xenograft tumor mouse model**

In the present example, in order to measure the drug (siRNA) delivery efficiency by the lipid nanoparticle according to one example, the change in the tumor size upon knock-down of PMVK known as a factor related to radiotherapy resistance in a pancreatic cancer xenograft mouse model was measured.

$3\times10^6$ Miapaca-2 cancer cells (ATCC, Cat. No. CRL-1420) and $1\times10^6$ A549 cancer cells (ATCC, Cat. No. CCL-185) were subcutaneously inoculated into BALB/c male 5-week-old nude mice (Orient Bio) 20g mice right thighs. The mouse tumor volume and body weight were monitored three times a week. The tumor size was measured using digital calipers. The volume of the tumor was calculated using $V = 0.5 \times W^2 \times L$ (V = tumor volume, W = tumor width, L = tumor length). In a similar way to the method of Example 7-5, the 246-C10 lipid nanoparticles (comprising lipid-PEG of 1 mol%) in which siRNA (SEQ ID Nos: 14 and 15; Table 3) targeting a PMVK gene was inoculated were prepared. When the tumor size reached $100mm^3 \pm 20$, the PMVK-encapsulated 246-C10 lipid nanoparticle and vehicle LNP were injected by IV (Intravenouse) at 3mg/kg as QD $\times$ 3 in the first week and the third week in the Miapaca-2 Xenograft nude mice, and after injection of the nanoparticle, once a week, using a 6-MV photon beam linear accelerator, radiation of 4 Gy was treated. The result was shown in FIG. 7f.

In a similar way to the method of Example 7-5, the 246-C10 lipid nanoparticles (comprising lipid-PEG of 1 mol%) in which siRNA (SEQ ID Nos: 14 and 15; Table 3) targeting a PMVK gene was inoculated were prepared. When the tumor size reached $100mm^3 \pm 20$, the PMVK-encapsulated LNP, and vehicle LNP were injected by IV (Intravenouse) at 3mg/kg as QD $\times$ 3 in the first week and the third week in the Miapaca-2 Xenograft nude mice, and in addition, after injection of the nanoparticle, once a week, using a 6-MV photon beam linear accelerator, radiation of 2 Gy was treated. The result was shown in FIG. 7g. As shown in FIG. 7f and FIG. 7g, it could be confirmed that the decrease in expression of the PMVK gene in the mouse model in which the 246-C10 lipid nanoparticle comprising Anti-PMKV siRNA was administered was further reduced when radiation was treated.

## Claims

1. A pharmaceutical composition for preventing or treating cancer,

comprising (1) a lipid nanoparticle comprising an ionizable lipid in which 1,4-bis(3-aminopropyl)piperazine and

alkyl-epoxide are bonded; a phospholipid; cholesterol; and a lipid-PEG (polyethyleneglycol) conjugate and
(2) a first anticancer agent,
wherein the first anticancer agent is an anionic drug, a nucleic acid, or a combination thereof.

**2.** The pharmaceutical composition according to claim 1, wherein the alkyl-epoxide is 1,2-epoxydodecane.

**3.** The pharmaceutical composition according to claim 1, wherein the phospholipid is one or more kinds selected from the group consisting of DOPE, DSPC, POPC, EPC, DOPC, DPPC, DOPG, DPPG, DSPE, Phosphatidylethanolamine, dipalmitoylphosphatidylethanolamine, 1,2-dioleoyl-sn-glycero-3-phosphoethanolamine, POPE, DOPS, and 1,2-dioleoyl-sn-glycero-3-[phospho-L-serine].

**4.** The pharmaceutical composition according to claim 1, wherein the lipid-PEG conjugate is one or more kinds selected from the group consisting of ceramide, dimyristoylglycerol (DMG), succinoyl-diacylglycerol (s-DAG), distearoylphosphatidylcholine (DSPC), distearoylphosphatidylethanolamine (DSPE), and cholesterol.

**5.** The pharmaceutical composition according to claim 1, wherein the lipid-PEG conjugate is comprised in 0.25 to 10 mol%.

**6.** The pharmaceutical composition according to claim 1, wherein the lipid nanoparticle comprises the ionizable lipid : phospholipid : cholesterol : lipid-PEG conjugate at a molar ratio of 20 to 50 : 10 to 30 : 10 to 60 : 0.25 to 10.

**7.** The pharmaceutical composition according to claim 1, wherein the lipid nanoparticle has a pKa of 6.0 to 7.0.

**8.** The pharmaceutical composition according to claim 1, wherein the first anticancer agent is encapsulated inside of the lipid nanoparticle.

**9.** The pharmaceutical composition according to claim 1, wherein the lipid nanoparticle has an average diameter of 30nm to 150nm.

**10.** The pharmaceutical composition according to claim 1, wherein the anionic drug is one or more kinds selected from the group consisting of a peptide, a protein drug, a protein-nucleic acid structure, and an anionic biopolymer-drug conjugate.

**11.** The pharmaceutical composition according to claim 1, wherein the nucleic acid is one or more kinds selected from the group consisting of small interfering ribonucleic acid (siRNA), ribosome ribonucleic acid (rRNA), ribonucleic acid (RNA), deoxyribonucleic acid (DNA), complementary deoxyribonucleic acid (cDNA), aptamer, messenger ribonucleic acid (mRNA), transfer ribonucleic acid (tRNA), antisense oligonucleotide, shRNA, miRNA, ribozyme, PNA and DNAzyme.

**12.** The pharmaceutical composition according to claim 1, wherein the cancer is cancer caused by HPV (human papilloma virus) infection.

**13.** The pharmaceutical composition according to claim 1, wherein the cancer is selected from the group consisting of cervical cancer, endometrial cancer, vagina cancer, vulvacancer, anal cancer, penis cancer, tonsil cancer, pharynx cancer, larynx cancer, head and neck cancer, squamous cell head and neck cancer, lung adenocarcinoma, non-small cell lung cancer, lung squamous cell carcinoma, lung cancer, stomach cancer, sarcoma, lymphoma, Hodgkin's disease, chronic or acute leukemia, thymic carcinoma, epithelial cancer, salivary gland cancer, liver cancer, thyroid cancer, parathyroid cancer, ovarian cancer, breast cancer, prostate cancer, esophageal cancer, pancreatic cancer, glioma, multiple myeloma, renal cell carcinoma, bladder cancer, choriocarcinoma, colon cancer, oral cancer, skin cancer, melanoma, bone cancer, rectal cancer, small intestine cancer, endocrine gland carcinoma, adrenal cancer, soft tissue sarcoma, urethral cancer, spinal cord tumor, brainstem glioma, and pituitary adenoma.

**14.** A pharmaceutical composition for treating cancer for use in combination with radiation therapy, comprising the pharmaceutical composition according to any one claim of claim 1 to claim 13.

**15.** A pharmaceutical composition for administration in combination with an anticancer agent, further comprising the pharmaceutical composition according to any one claim of claim 1 to claim 13 and a second anticancer agent.

16. The pharmaceutical composition for administration in combination with an anticancer agent according to claim 15, wherein the second anticancer agent is one or more kinds selected from the group consisting of cisplatin, paclitaxel, doxetaxel, gemcitabine, doxorubicin, fluorouracil and carboplatin.

【FIG. 1a】

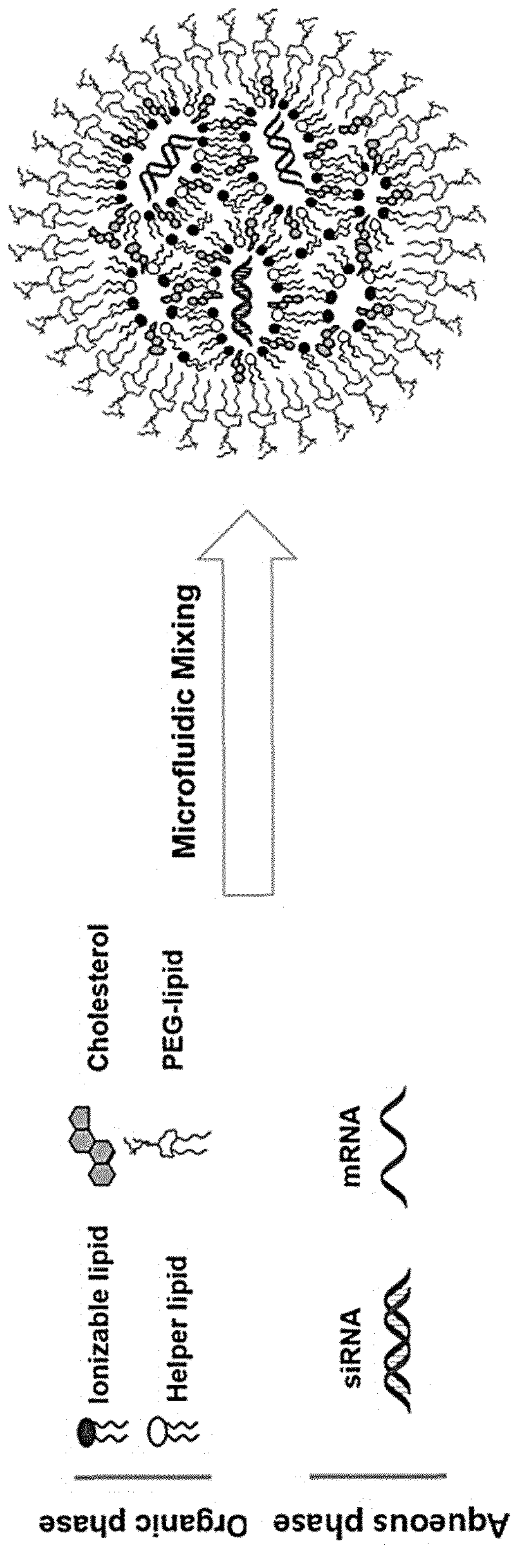
Microfluidic Mixing
Cholesterol
PEG-lipid
Ionizable lipid
Helper lipid
mRNA
siRNA
Organic phase
Aqueous phase

【FIG. 1b】

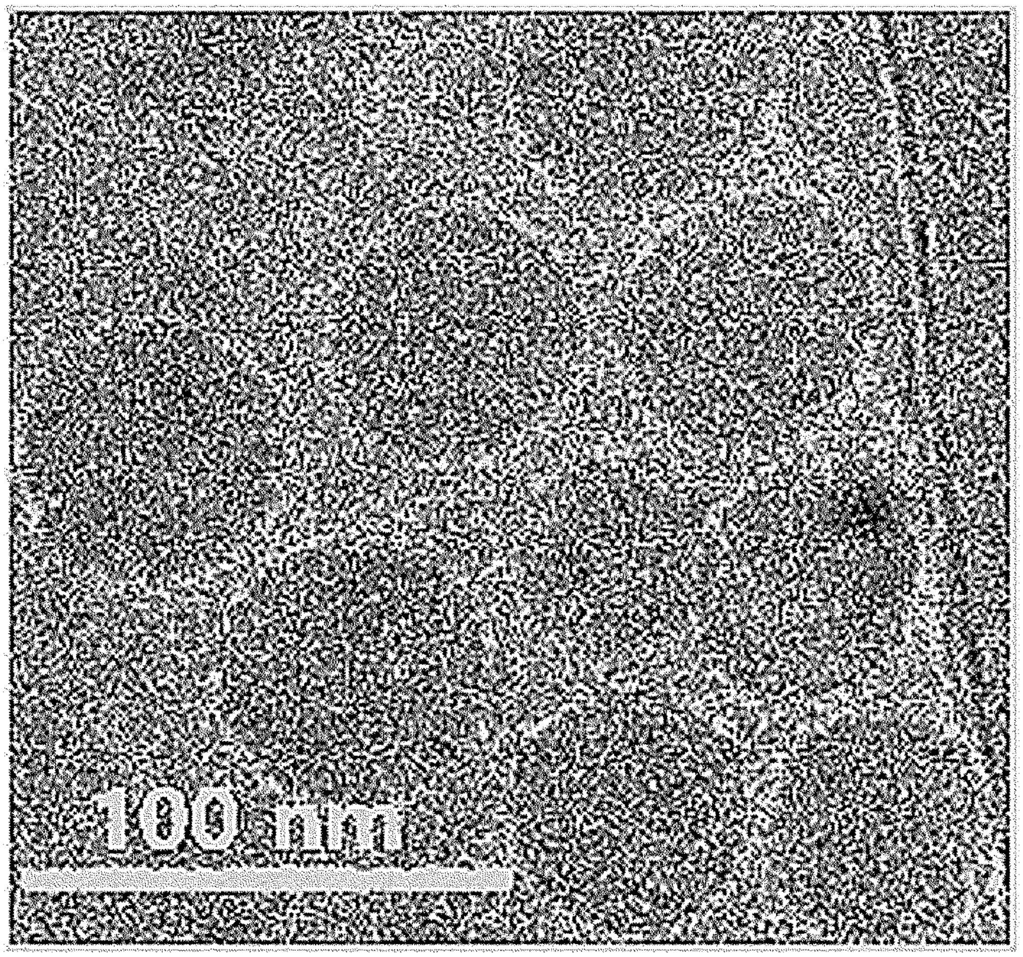

【FIG. 2】

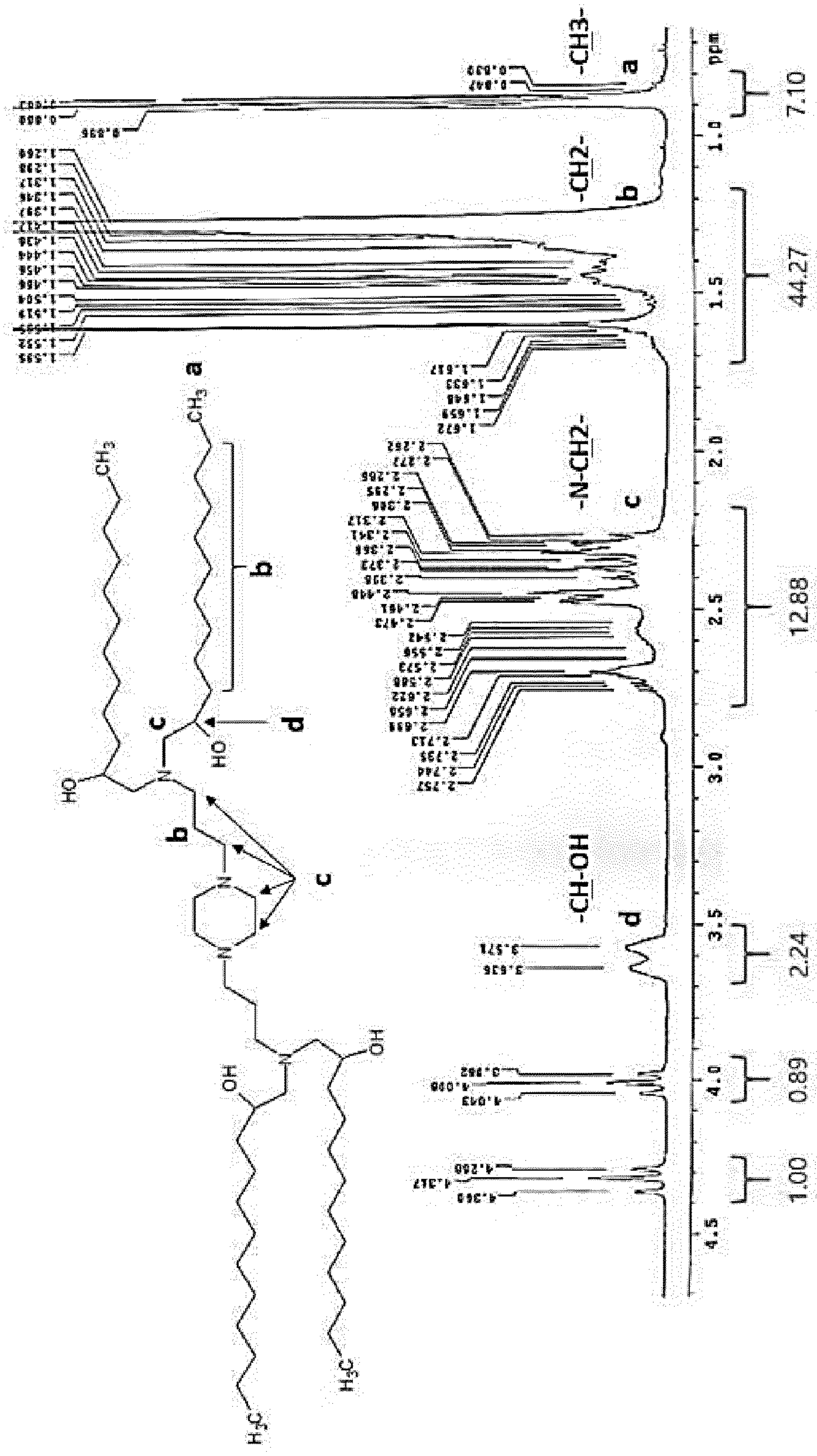


Room temperature 400 MHz 1H NMR spectrum of 246C10 in CDCl3.

【FIG. 3a】

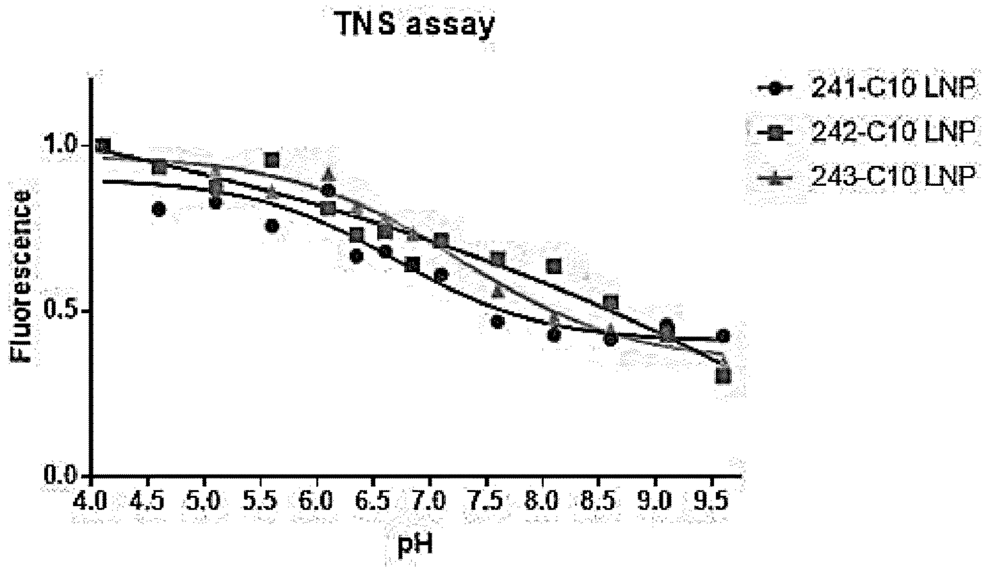
TNS assay
241-C10 LNP
242-C10 LNP
243-C10 LNP
Fluorescence
1.0
0.5
0.0
4.0
4.5
5.0
5.5
6.0
6.5
7.0
7.5
8.0
8.5
9.0
9.5
pH

【FIG. 3b】

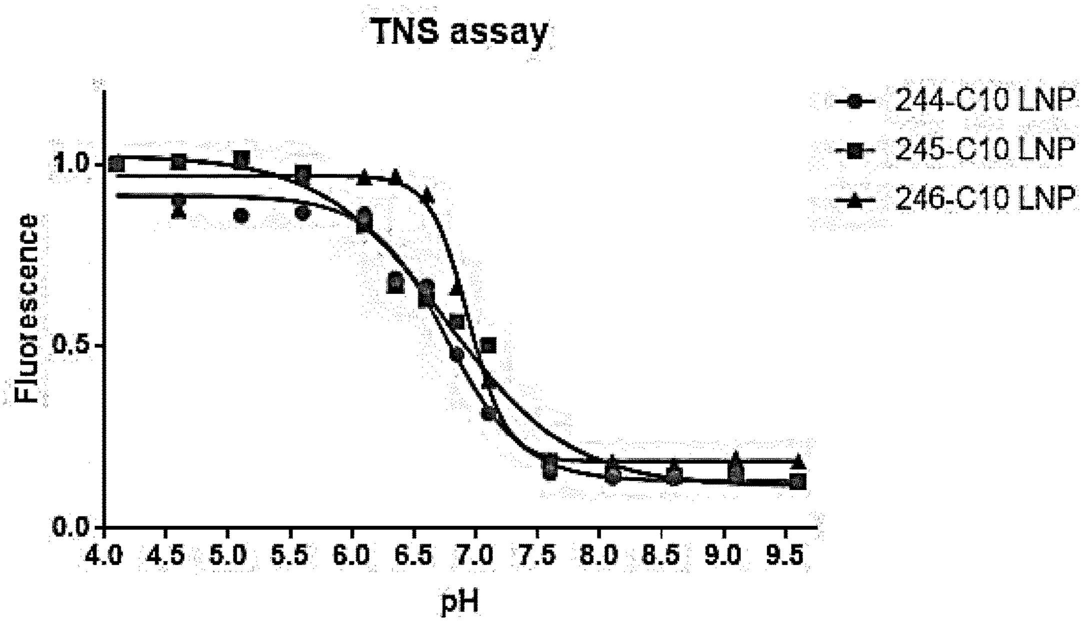
TNS assay
Fluorescence
1.0
0.5
0.0
4.0
4.5
5.0
5.5
6.0
6.5
7.0
7.5
8.0
8.5
9.0
9.5
pH
244-C10 LNP
245-C10 LNP
246-C10 LNP

【FIG. 4a】

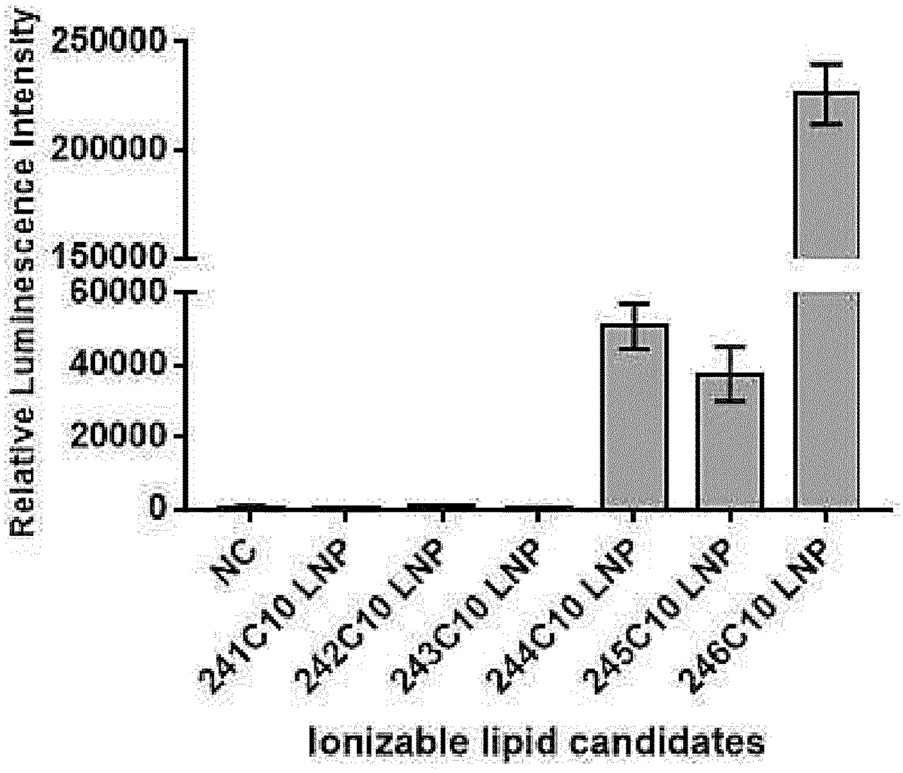

Relative Luminescence Intensity
250000
200000
150000
60000
40000
20000
0
NC
241C10 LNP
242C10 LNP
243C10 LNP
244C10 LNP
245C10 LNP
246C10 LNP
Ionizable lipid candidates

【FIG. 4b】

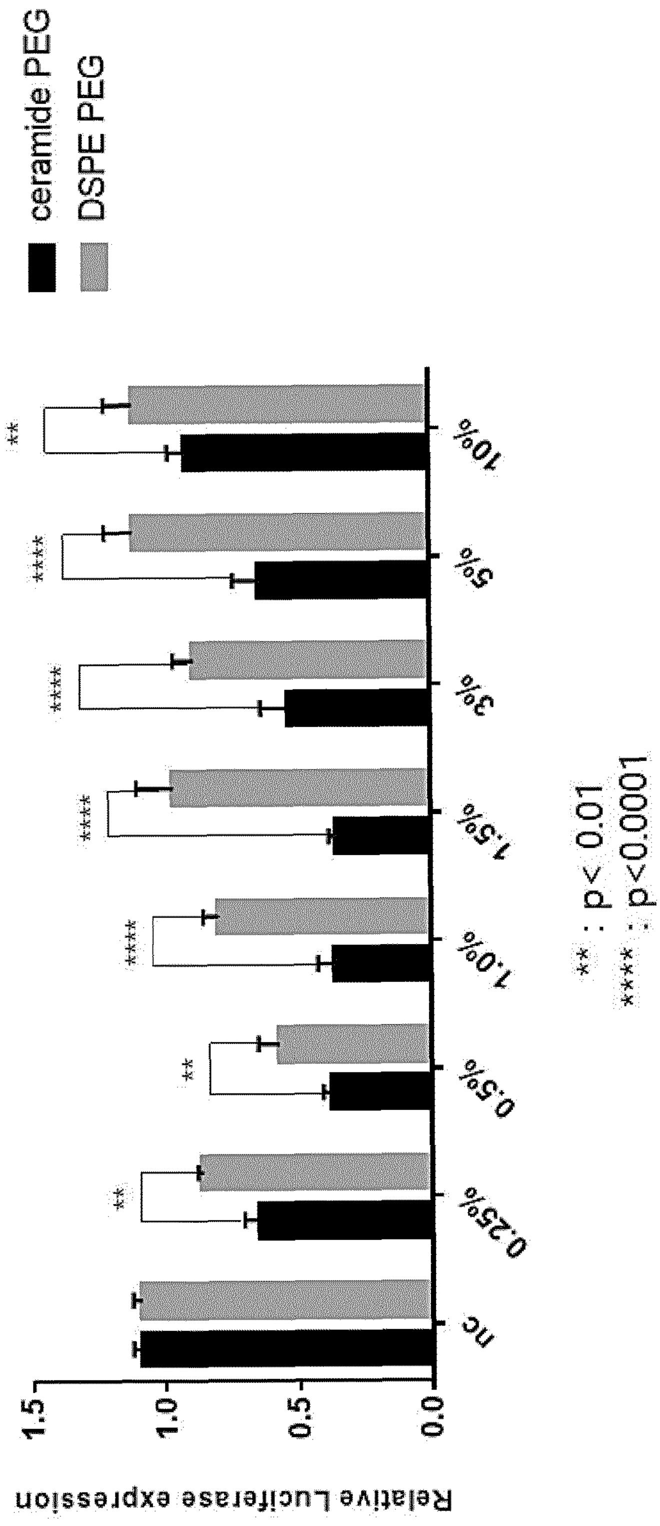
ceramide PEG
DSPE PEG
Relative Luciferase expression
1.5
1.0
0.5
0.0
nc
0.25%
0.5%
1.0%
1.5%
3%
5%
10%
**
****
** : p< 0.01
**** : p<0.0001

【FIG. 5a】

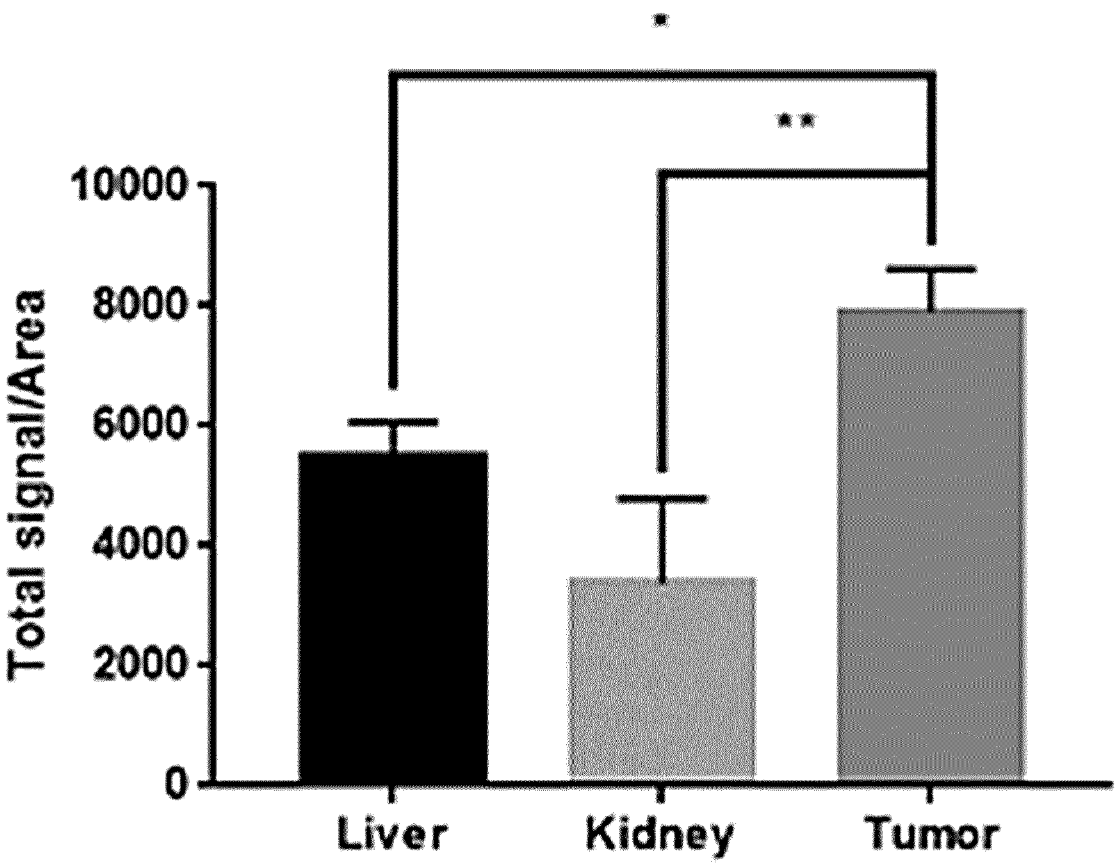
*
**
10000
8000
6000
4000
2000
0
Total signal/Area
Liver
Kidney
Tumor

【FIG. 5b】

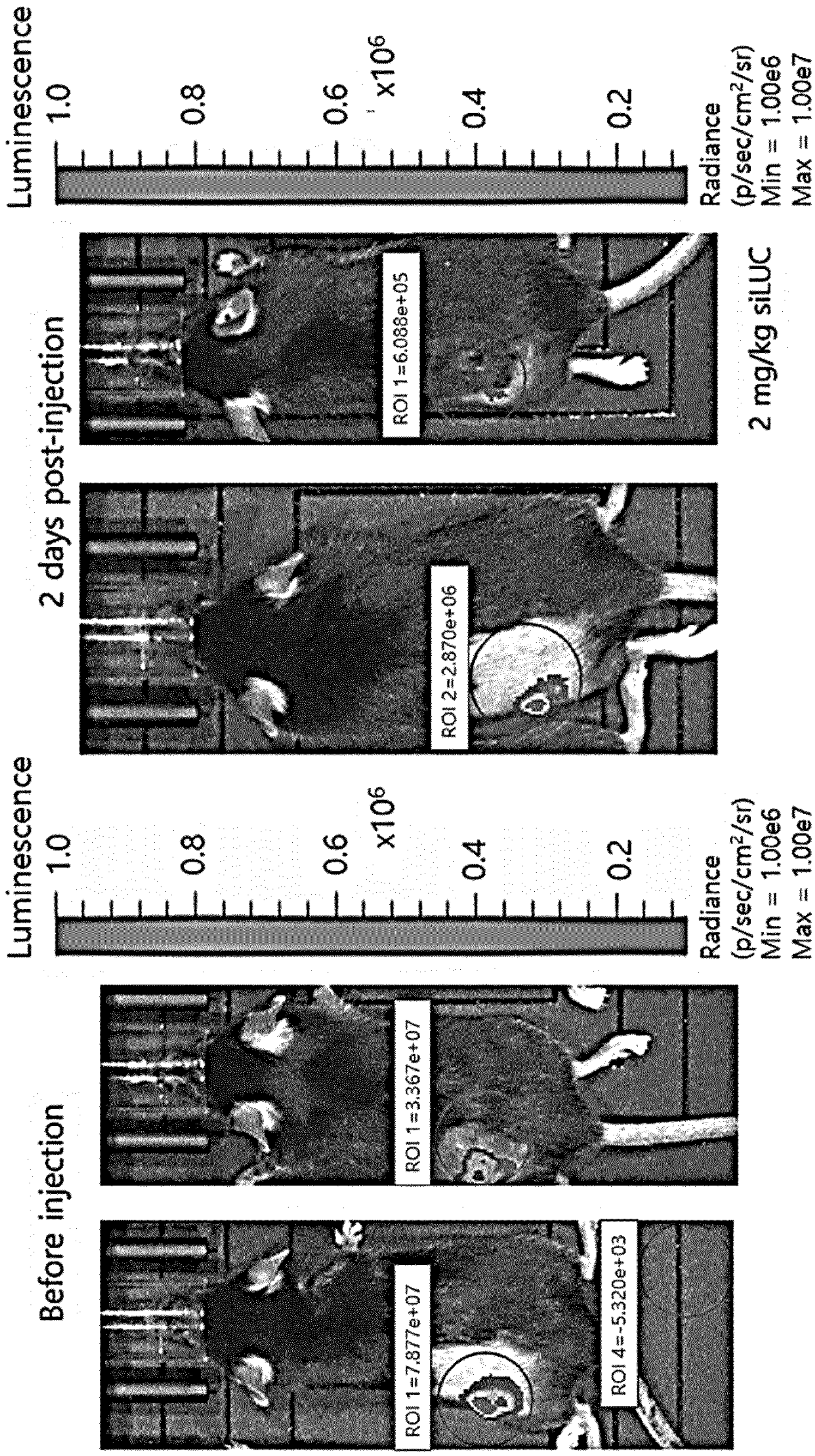
Before injection
2 days post-injection
2 mg/kg siLUC
ROI 1=7.877e+07
ROI 4=-5.320e+03
ROI 1=3.367e+07
ROI 2=2.870e+06
ROI 1=6.088e+05
Luminescence
1.0
0.8
0.6
0.4
0.2
x10^6
Radiance
(p/sec/cm^2/sr)
Min = 1.00e6
Max = 1.00e7

【FIG. 5c】

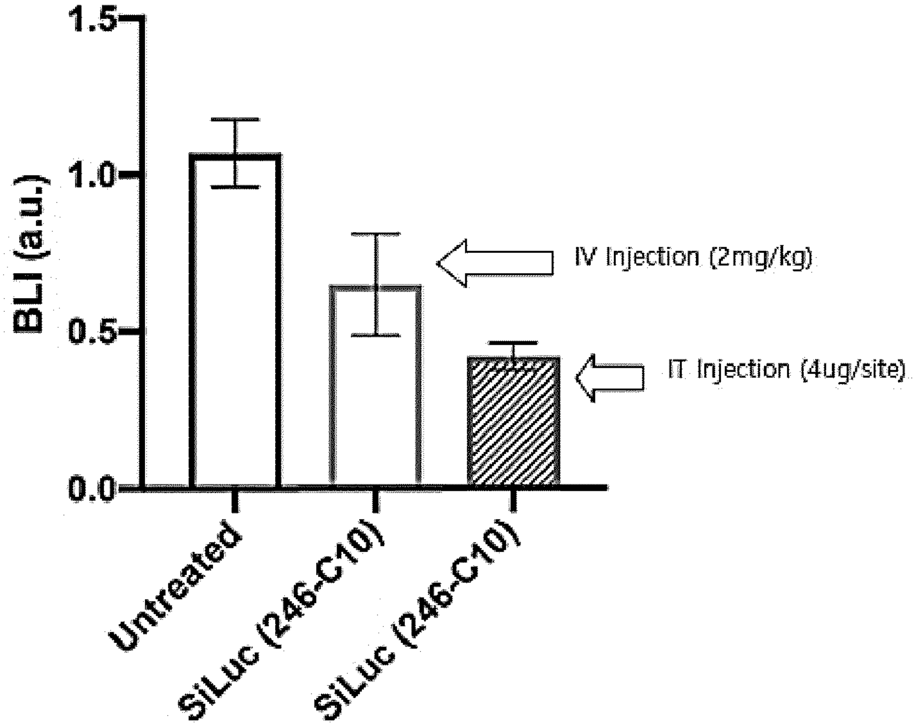
1.5
1.0
0.5
0.0
BLI (a.u.)
IV Injection (2mg/kg)
IT Injection (4ug/site)
Untreated
SiLuc (246-C10)
SiLuc (246-C10)

【FIG. 6a】

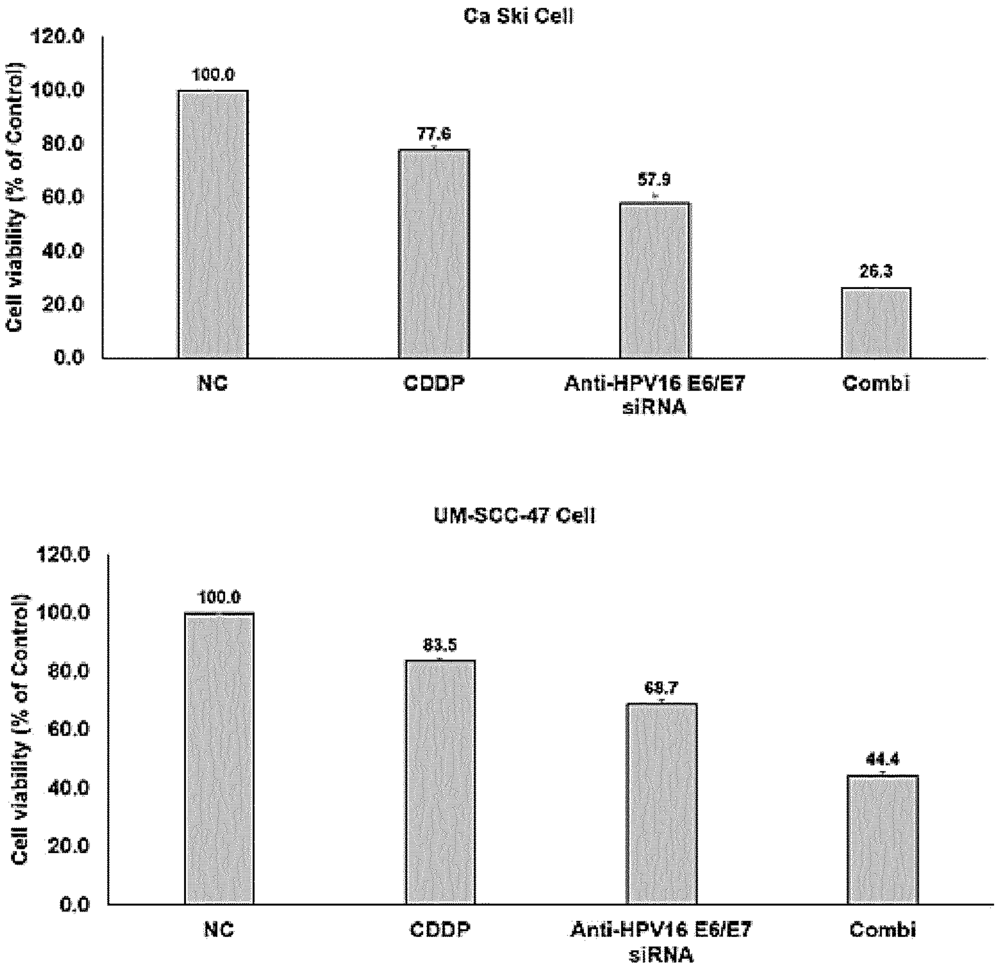
Ca Ski Cell
Cell viability (% of Control)
120.0
100.0
80.0
60.0
40.0
20.0
0.0
100.0
77.6
57.9
26.3
NC
CDDP
Anti-HPV16 E6/E7 siRNA
Combi
UM-SCC-47 Cell
Cell viability (% of Control)
120.0
100.0
80.0
60.0
40.0
20.0
0.0
100.0
83.5
68.7
44.4
NC
CDDP
Anti-HPV16 E6/E7 siRNA
Combi

【FIG. 6b】

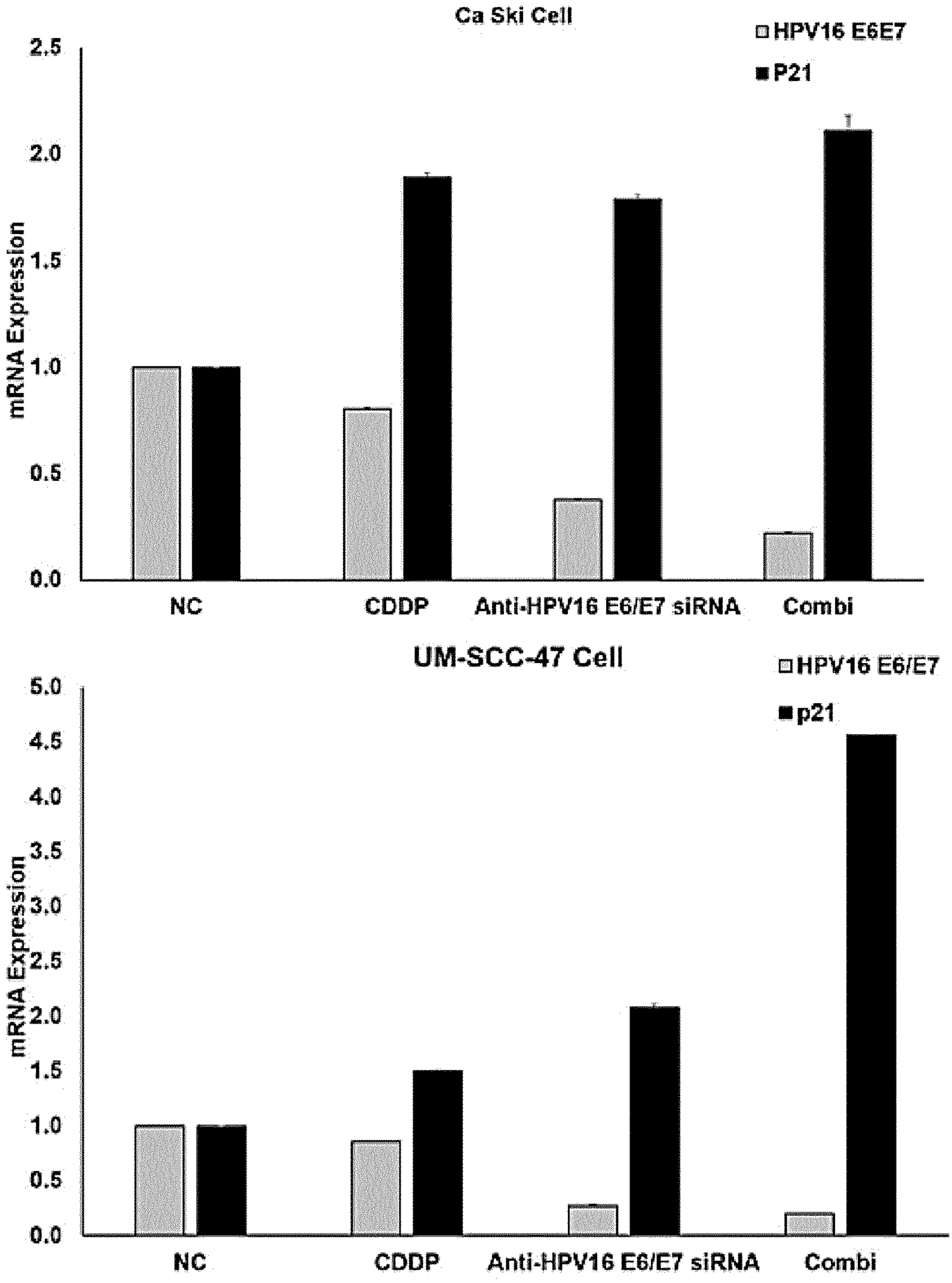
Ca Ski Cell
HPV16 E6E7
P21
mRNA Expression
2.5
2.0
1.5
1.0
0.5
0.0
NC
CDDP
Anti-HPV16 E6/E7 siRNA
Combi
UM-SCC-47 Cell
HPV16 E6/E7
p21
mRNA Expression
5.0
4.5
4.0
3.5
3.0
2.5
2.0
1.5
1.0
0.5
0.0
NC
CDDP
Anti-HPV16 E6/E7 siRNA
Combi

【FIG. 6c】

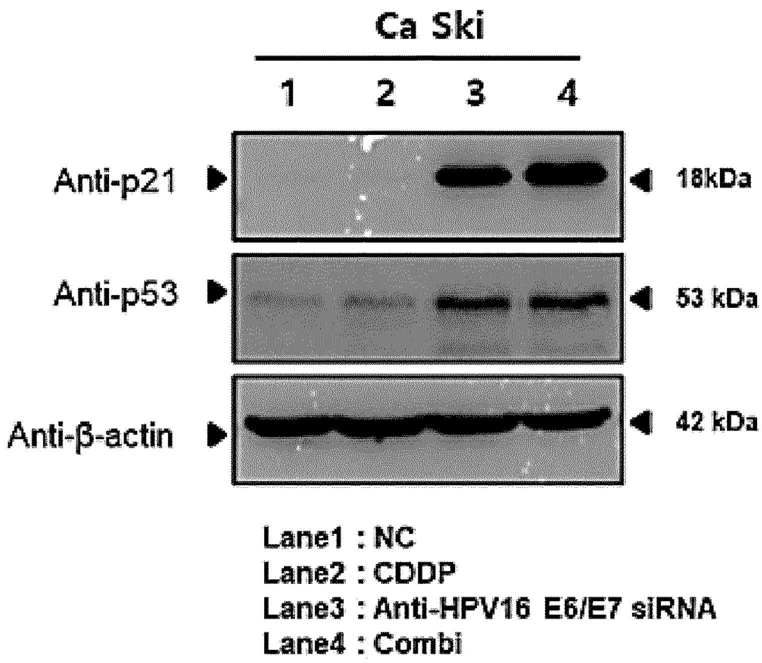
Ca Ski
1
2
3
4
Anti-p21
Anti-p53
Anti-β-actin
18kDa
53 kDa
42 kDa
Lane1 : NC
Lane2 : CDDP
Lane3 : Anti-HPV16 E6/E7 siRNA
Lane4 : Combi

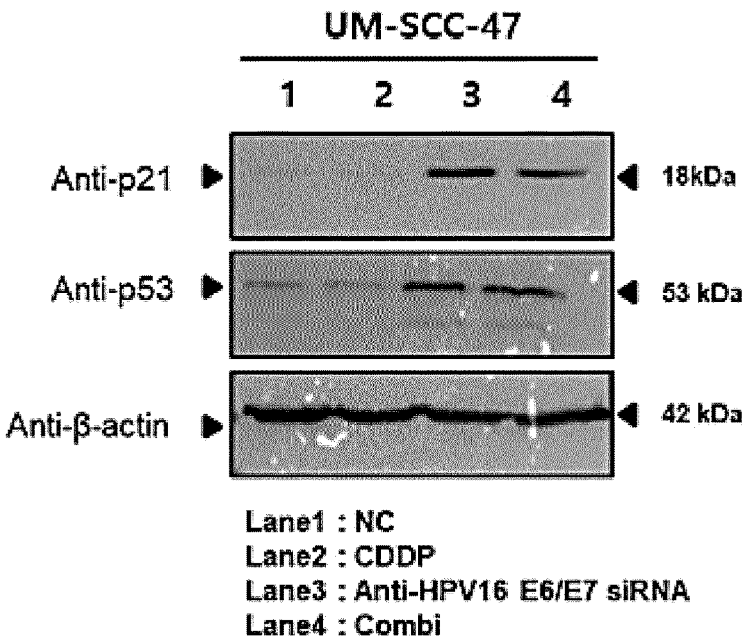
UM-SCC-47
1
2
3
4
Anti-p21
Anti-p53
Anti-β-actin
18kDa
53 kDa
42 kDa
Lane1 : NC
Lane2 : CDDP
Lane3 : Anti-HPV16 E6/E7 siRNA
Lane4 : Combi

【FIG. 7a】

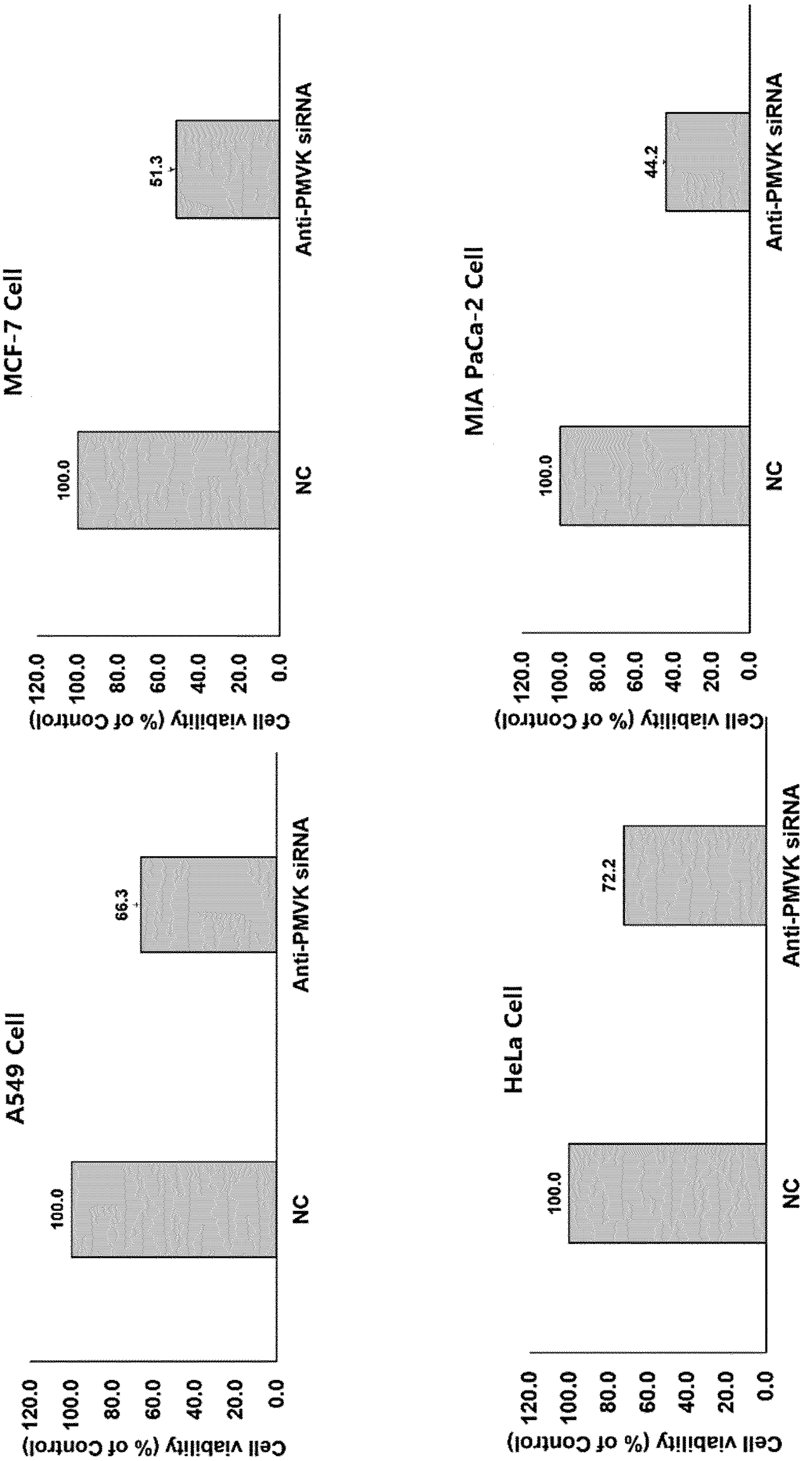
MCF-7 Cell
Cell viability (% of Control)
120.0
100.0
80.0
60.0
40.0
20.0
0.0
100.0
51.3
NC
Anti-PMVK siRNA
MIA PaCa-2 Cell
Cell viability (% of Control)
120.0
100.0
80.0
60.0
40.0
20.0
0.0
100.0
44.2
NC
Anti-PMVK siRNA
A549 Cell
Cell viability (% of Control)
120.0
100.0
80.0
60.0
40.0
20.0
0.0
100.0
66.3
NC
Anti-PMVK siRNA
HeLa Cell
Cell viability (% of Control)
120.0
100.0
80.0
60.0
40.0
20.0
0.0
100.0
72.2
NC
Anti-PMVK siRNA

【FIG. 7b】

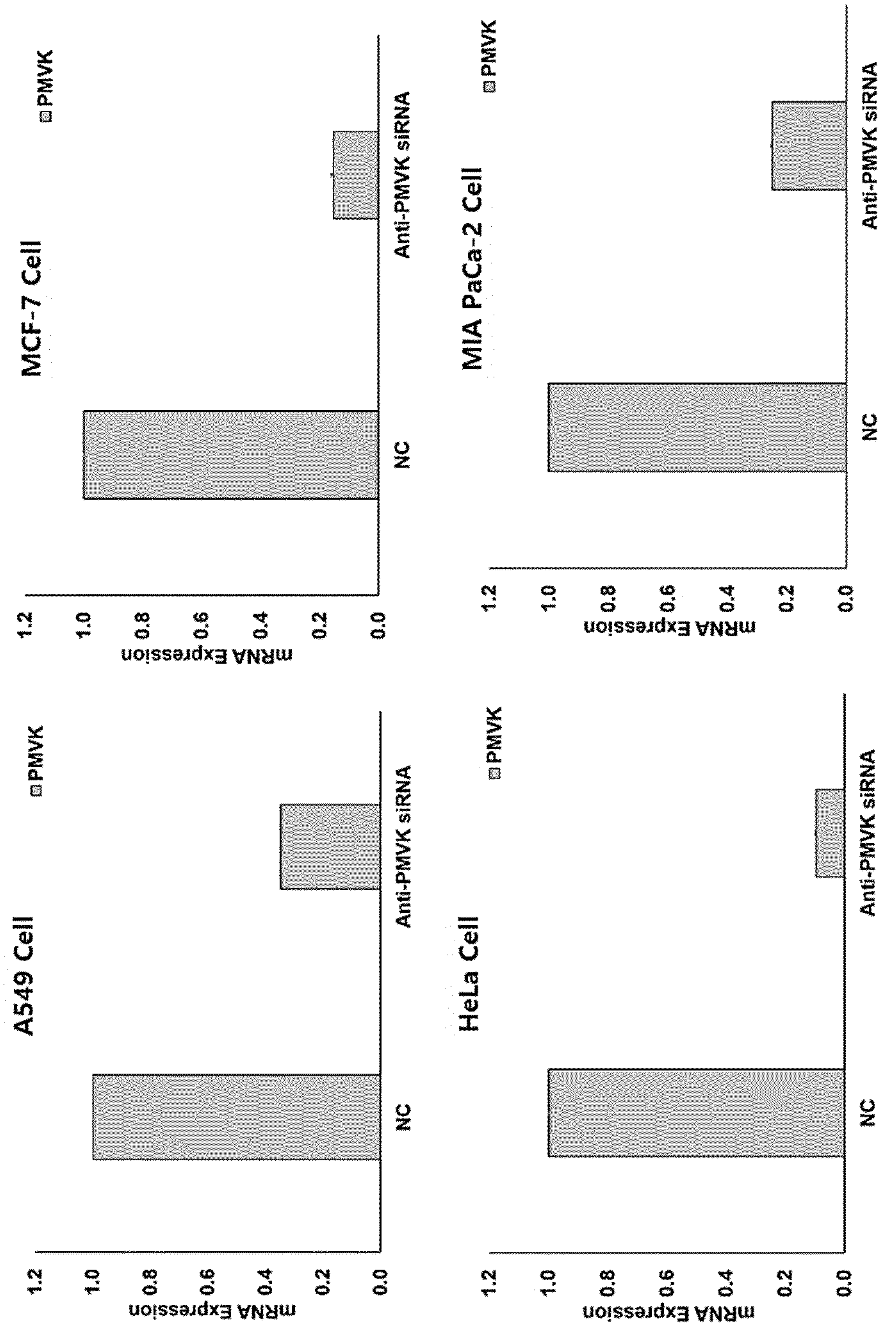
MCF-7 Cell
PMVK
mRNA Expression
1.2
1.0
0.8
0.6
0.4
0.2
0.0
NC
Anti-PMVK siRNA
MIA PaCa-2 Cell
PMVK
mRNA Expression
1.2
1.0
0.8
0.6
0.4
0.2
0.0
NC
Anti-PMVK siRNA
A549 Cell
PMVK
mRNA Expression
1.2
1.0
0.8
0.6
0.4
0.2
0.0
NC
Anti-PMVK siRNA
HeLa Cell
PMVK
mRNA Expression
1.2
1.0
0.8
0.6
0.4
0.2
0.0
NC
Anti-PMVK siRNA

【FIG. 7c】

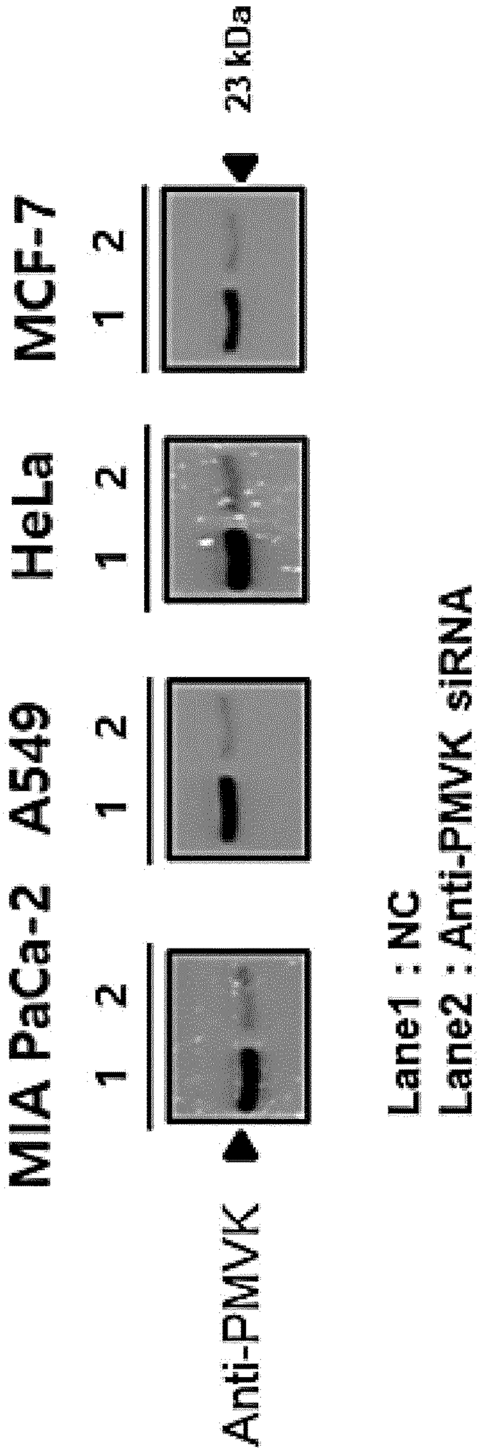
MIA PaCa-2
A549
HeLa
MCF-7
1 2
1 2
1 2
1 2
Anti-PMVK
23 kDa
Lane1 : NC
Lane2 : Anti-PMVK siRNA

【FIG. 7d】

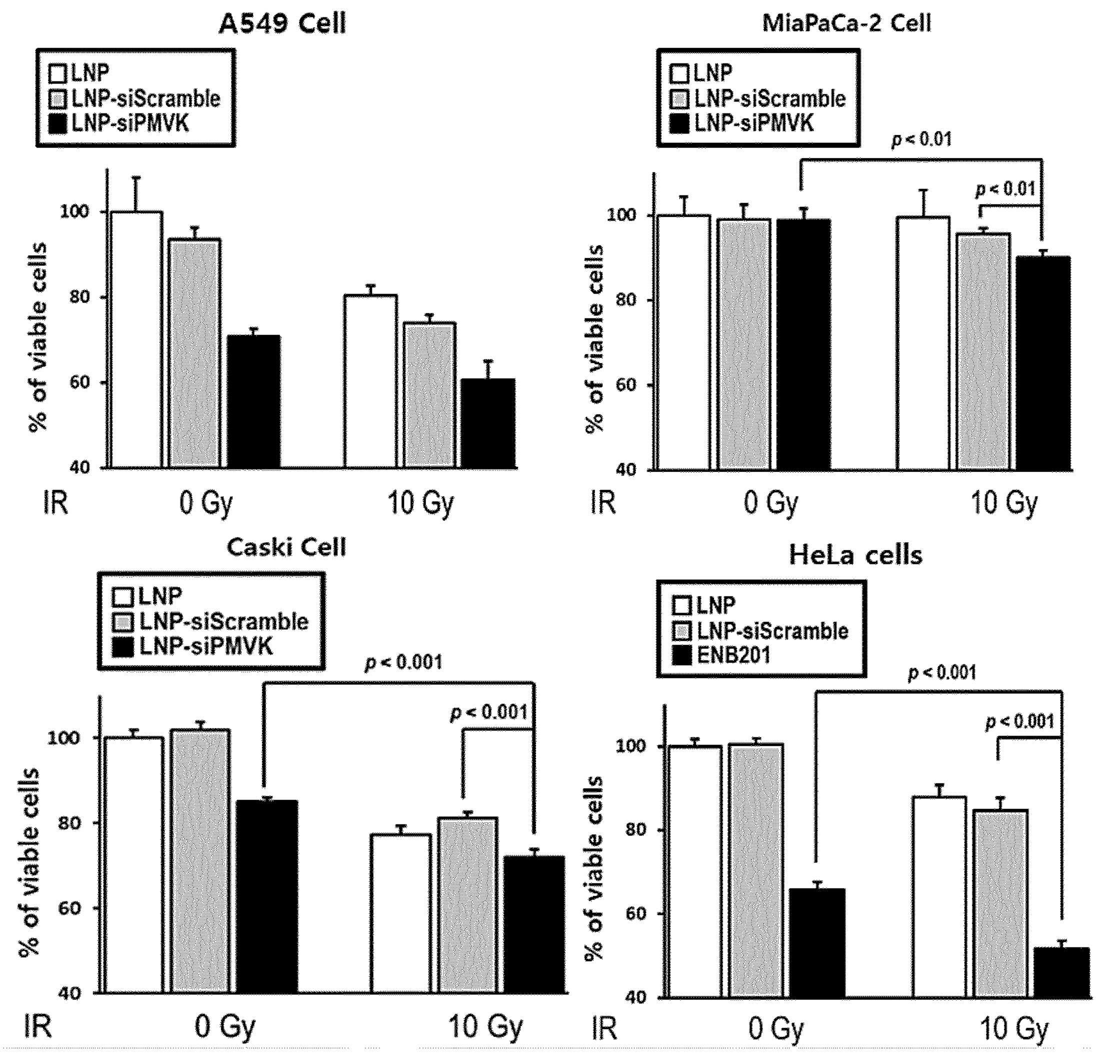
A549 Cell
LNP
LNP-siScramble
LNP-siPMVK
% of viable cells
100
80
60
40
IR
0 Gy
10 Gy
MiaPaCa-2 Cell
LNP
LNP-siScramble
LNP-siPMVK
$p < 0.01$
$p < 0.01$
% of viable cells
100
80
60
40
IR
0 Gy
10 Gy
Caski Cell
LNP
LNP-siScramble
LNP-siPMVK
$p < 0.001$
$p < 0.001$
% of viable cells
100
80
60
40
IR
0 Gy
10 Gy
HeLa cells
LNP
LNP-siScramble
ENB201
$p < 0.001$
$p < 0.001$
% of viable cells
100
80
60
40
IR
0 Gy
10 Gy

【FIG. 7e】

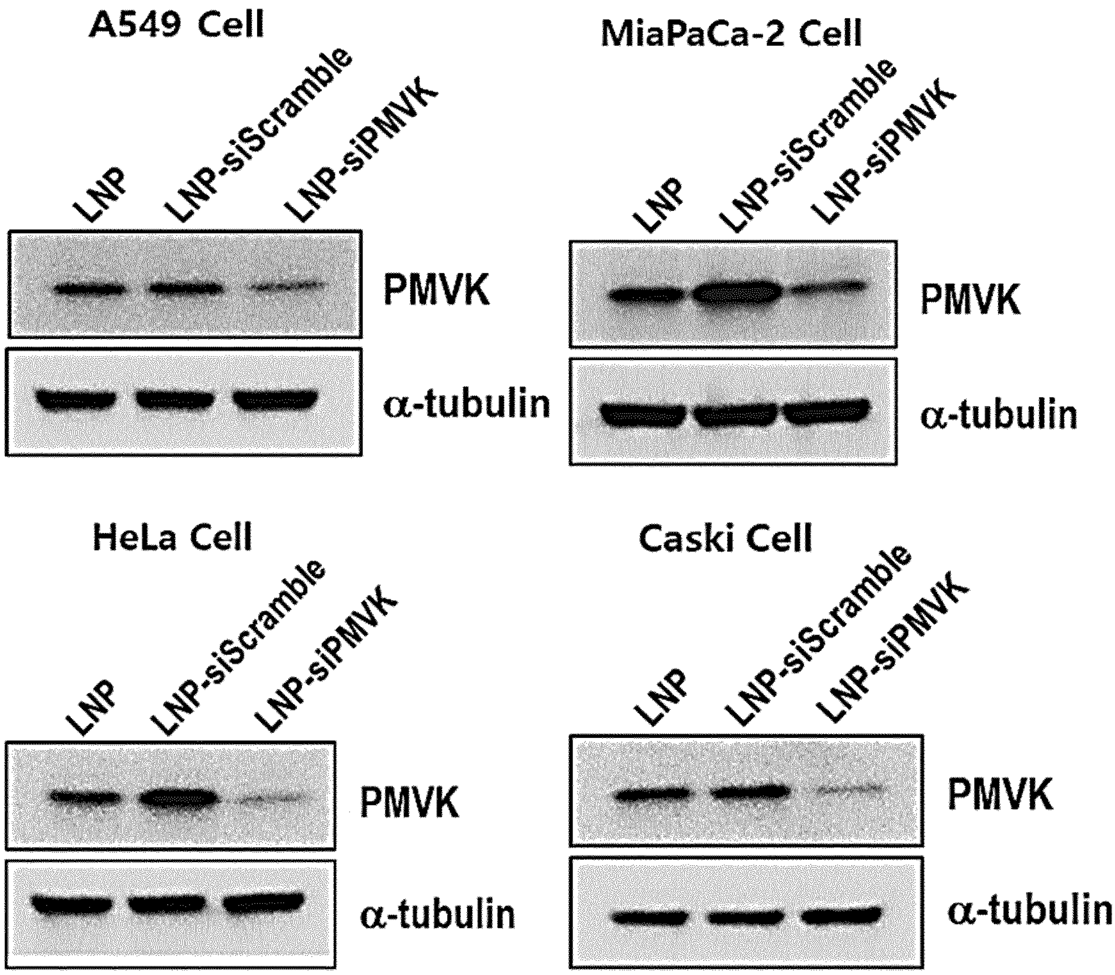
A549 Cell
LNP
LNP-siScramble
LNP-siPMVK
PMVK
α-tubulin
MiaPaCa-2 Cell
LNP
LNP-siScramble
LNP-siPMVK
PMVK
α-tubulin
HeLa Cell
LNP
LNP-siScramble
LNP-siPMVK
PMVK
α-tubulin
Caski Cell
LNP
LNP-siScramble
LNP-siPMVK
PMVK
α-tubulin

【FIG. 7f】

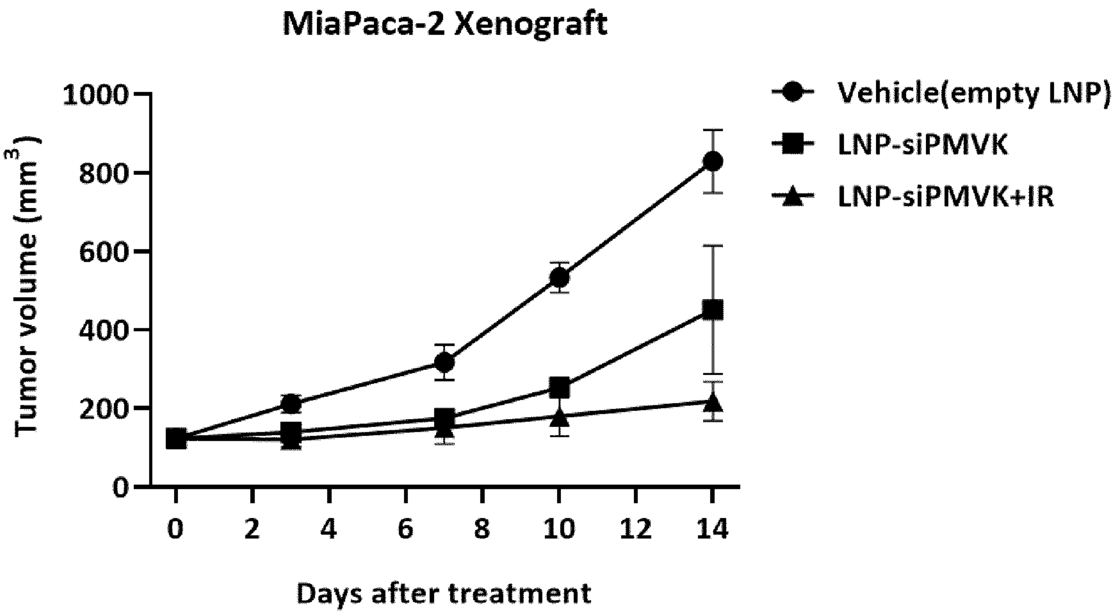
MiaPaca-2 Xenograft
Tumor volume (mm3)
1000
800
600
400
200
0
0
2
4
6
8
10
12
14
Days after treatment
Vehicle(empty LNP)
LNP-siPMVK
LNP-siPMVK+IR

【FIG. 7g】

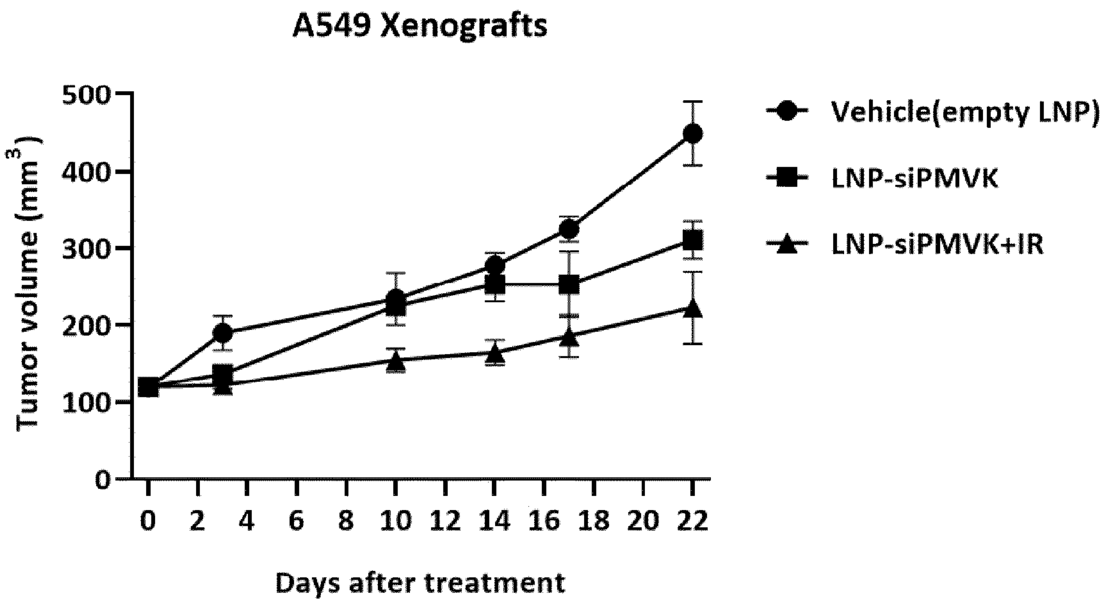
A549 Xenografts
Tumor volume (mm3)
500
400
300
200
100
0
0 2 4 6 8 10 12 14 16 18 20 22
Days after treatment
Vehicle(empty LNP)
LNP-siPMVK
LNP-siPMVK+IR

## INTERNATIONAL SEARCH REPORT

International application No.
**PCT/KR2021/019773**

### A. CLASSIFICATION OF SUBJECT MATTER

**A61K 48/00**(2006.01)i; **A61K 9/51**(2006.01)i; **A61K 31/7088**(2006.01)i; **A61P 35/00**(2006.01)i; **A61K 45/06**(2006.01)i

According to International Patent Classification (IPC) or to both national classification and IPC

### B. FIELDS SEARCHED

Minimum documentation searched (classification system followed by classification symbols)

A61K 48/00(2006.01); A61K 45/08(2006.01); A61K 47/18(2006.01); A61K 47/28(2006.01); A61K 9/19(2006.01); A61K 9/51(2006.01); B82B 3/00(2006.01); C01G 19/02(2006.01); C07C 217/42(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 지질-PEG(lipid-PEG), 1,4-비스(3-아미노프로필) 피페라진(1,4-bis(3-aminopropyl)piperazine), 핵산(nucleic acid), siRNA, 나노입자(nanoparticle), 약물(drug), 항암(anticancer)

### C. DOCUMENTS CONSIDERED TO BE RELEVANT

| Category* | Citation of document, with indication, where appropriate, of the relevant passages | Relevant to claim No. |
|---|---|---|
| X | WO 2019-110067 A1 (AARHUS UNIVERSITET) 13 June 2019 (2019-06-13)<br>See claims 1, 7-9 and 15; page 2, lines 10-11 and 24-28; page 27, lines 17-22; and figure 3A. | 1-13 |
| Y | | 14-16 |
| Y | KR 10-2008-0040412 A (SEOUL NATIONAL UNIVERSITY INDUSTRY FOUNDATION) 08 May 2008 (2008-05-08)<br>See claims 1, 4, 6-7 and 12; and paragraphs [0042], [0054] and [0074]. | 14-16 |
| A | LOVE, K. T. et al. Lipid-like materials for low-dose, in vivo gene silencing. PNAS. February 2010, vol. 107, no. 5, pp. 1864-1869.<br>See abstract; and figures 1-6. | 1-16 |
| A | WO 2013-188763 A1 (THE BRIGHAM AND WOMEN`S HOSPITAL, INC.) 19 December 2013 (2013-12-19)<br>See entire document. | 1-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 April 2022** | **05 April 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No.
**PCT/KR2021/019773**

**C. DOCUMENTS CONSIDERED TO BE RELEVANT**

| Category* | Citation of document, with indication, where appropriate, of the relevant passages | Relevant to claim No. |
|---|---|---|
| A | WO 2010-053572 A2 (MASSACHUSETTS INSTITUTE OF TECHNOLOGY) 14 May 2010 (2010-05-14)<br>See entire document. | 1-16 |

Form PCT/ISA/210 (second sheet) (July 2019)

**INTERNATIONAL SEARCH REPORT**
**Information on patent family members**

International application No.
**PCT/KR2021/019773**

| Patent document cited in search report | Publication date (day/month/year) | Patent family member(s) | Publication date (day/month/year) |
|---|---|---|---|
| WO 2019-110067 A1 | 13 June 2019 | None | |
| KR 10-2008-0040412 A | 08 May 2008 | KR 10-0962301 B1 | 11 June 2010 |
| | | US 2010-0062051 A1 | 11 March 2010 |
| | | US 8377899 B2 | 19 February 2013 |
| | | WO 2008-054184 A1 | 08 May 2008 |
| WO 2013-188763 A1 | 19 December 2013 | AU 2013-274101 A1 | 29 January 2015 |
| | | AU 2013-274101 B2 | 07 September 2017 |
| | | AU 2017-265136 A1 | 04 January 2018 |
| | | BR 112014031421 A2 | 27 June 2017 |
| | | CN 104582732 A | 29 April 2015 |
| | | EP 2861256 A1 | 22 April 2015 |
| | | EP 2861256 A4 | 01 June 2016 |
| | | EP 2861256 B1 | 23 October 2019 |
| | | JP 2015-527301 A | 17 September 2015 |
| | | JP 2018-188443 A | 29 November 2018 |
| | | JP 6427097 B2 | 21 November 2018 |
| | | US 10300143 B2 | 28 May 2019 |
| | | US 2015-0174263 A1 | 25 June 2015 |
| | | US 2018-0125987 A1 | 10 May 2018 |
| | | US 9789193 B2 | 17 October 2017 |
| | | WO 2013-188763 A9 | 19 December 2013 |
| WO 2010-053572 A2 | 14 May 2010 | AU 2009-311667 A1 | 23 June 2011 |
| | | AU 2009-311667 B2 | 14 April 2016 |
| | | CA 2742954 A1 | 14 May 2010 |
| | | CA 2742954 C | 10 July 2018 |
| | | CA 3006395 A1 | 14 May 2010 |
| | | CN 102245559 A | 16 November 2011 |
| | | CN 102245559 B | 27 May 2015 |
| | | CN 104910025 A | 16 September 2015 |
| | | CN 104910025 B | 16 July 2019 |
| | | EP 2365962 A2 | 21 September 2011 |
| | | EP 2365962 A4 | 14 August 2013 |
| | | EP 2365962 B1 | 05 July 2017 |
| | | EP 3269395 A1 | 17 January 2018 |
| | | ES 2646630 T3 | 14 December 2017 |
| | | JP 2012-508235 A | 05 April 2012 |
| | | JP 2017-061563 A | 30 March 2017 |
| | | JP 2019-048839 A | 28 March 2019 |
| | | JP 2020-063257 A | 23 April 2020 |
| | | JP 6087504 B2 | 01 March 2017 |
| | | JP 6431027 B2 | 28 November 2018 |
| | | JP 6637141 B2 | 29 January 2020 |
| | | JP 6902287 B2 | 14 July 2021 |
| | | KR 10-1734955 B1 | 12 May 2017 |
| | | KR 10-2011-0087311 A | 02 August 2011 |
| | | MX 2011004859 A | 03 August 2011 |
| | | MX 353900 B | 01 February 2018 |
| | | US 10189802 B2 | 29 January 2019 |
| | | US 10844028 B2 | 24 November 2020 |
| | | US 2010-0331234 A1 | 30 December 2010 |

Form PCT/ISA/210 (patent family annex) (July 2019)

**INTERNATIONAL SEARCH REPORT**
**Information on patent family members**

International application No.
**PCT/KR2021/019773**

| Patent document cited in search report | Publication date (day/month/year) | Patent family member(s) | Publication date (day/month/year) |
|---|---|---|---|
| | | US 2011-0293703 A1 | 01 December 2011 |
| | | US 2015-0203439 A1 | 23 July 2015 |
| | | US 2017-0204075 A1 | 20 July 2017 |
| | | US 2019-0177289 A1 | 13 June 2019 |
| | | US 2021-0101875 A1 | 08 April 2021 |
| | | US 8450298 B2 | 28 May 2013 |
| | | US 8969353 B2 | 03 March 2015 |
| | | US 9556110 B2 | 31 January 2017 |
| | | WO 2010-053572 A9 | 14 May 2010 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **NOVINA ; SHARP.** *Nature,* 2004, vol. 430, 161-164
- **HIRKO et al.** *Curr. Med. Chem.,* 2003, vol. 10, 1185-93
- **MERDAN et al.** *Adv. Drug. Deliv. Rev.,* 2002, vol. 54, 715-58
- **SPAGNOU et al.** *Biochemistry,* 2004, vol. 43, 13348-13356
- **FILONAND ; PHILLIPS.** *Biochim. Biophys. Acta,* 1997, vol. 1329, 345-56
- **REN et al.** *Gene Therapy,* 2000, vol. 7, 764-768
- *CHEMICAL ABSTRACTS,* 7209-38-3
- **DE MESMAEKER et al.** *Curr Opin Struct Biol.,* 1995, vol. 5 (3), 343-55
- **NIELSEN PE ; EGHOLM M ; BERG RH ; BUCHARDT O.** Sequence-selective recognition of DNA by strand displacement with a thymine-substituted polyamide. *Science,* 1991, vol. 254, 1497-1500